# EUROPEAN PATENT SPECIFICATION

(11) **EP 3 261 681 B1**
(45) Date of publication and mention of the grant of the patent: **15.01.2020**
(21) Application number: 16707063.0
(22) Date of filing: 24.02.2016
(51) Int. Cl.: A61K 51/04, A61K 51/10

(54) **RADIOPHARMACEUTICAL SOLUTIONS WITH ADVANTAGEOUS PROPERTIES**
RADIOPHARMAZEUTISCHE LÖSUNGEN MIT VORTEILHAFTEN EIGENSCHAFTEN
SOLUTIONS RADIOPHARMACEUTIQUES AVEC DES PROPRIÉTÉS AVANTAGEUSES

(30) Priority: 26.02.2015 EP 15156714; 26.02.2015 US 201514632849
(43) Date of publication of application: 03.01.2018
(73) Proprietor: Sciencons AS, 0884 Oslo (NO)
(72) Inventor: LARSEN, Roy, Hartvig, 0598 Oslo (NO)
(74) Representative: Aera A/S
(86) International application number: PCT/EP2016/053874
(87) International publication number: WO 2016/135200

(56) References cited:
- AGATA PIOTROWSKA ET AL: "Functionalized NaA nanozeolites labeled with 224,225Ra for targeted alpha therapy", JOURNAL OF NANOPARTICLE RESEARCH, vol. 15, no. 11, 31 October 2013 (2013-10-31), XP055206059, ISSN: 1388-0764, DOI: 10.1007/s11051-013-2082-7
- MICHAEL D. DIENER ET AL: "212 Pb@C 60 and Its Water-Soluble Derivatives: Synthesis, Stability, and Suitability for Radioimmunotherapy", JOURNAL OF THE AMERICAN CHEMICAL SOCIETY, vol. 129, no. 16, 1 April 2007 (2007-04-01), pages 5131-5138, XP055205910, ISSN: 0002-7863, DOI: 10.1021/ja068639b
- JAGGI J S ET AL: "Efforts to Control the Errant Products of a Targeted in vivo Generator", CANCER RESEARCH, AMERICAN ASSOCIATION FOR CANCER RESEARCH, US, vol. 65, no. 11, 1 January 2005 (2005-01-01), pages 4888-4895, XP002998584, ISSN: 0008-5472, DOI: 10.1158/0008-5472.CAN-04-3096 cited in the application
- JONES S B ET AL: "Evaluation of Dithiol Chelating Agents as Potential Adjuvants for Anti-IL-2 Receptor Lead or Bismuth Alpha Radioimmunotherapy", NUCLEAR MEDICINE AND BIOLOGY, ELSEVIER, NY, US, vol. 23, no. 2, 1 February 1996 (1996-02-01), pages 105-113, XP004051672, ISSN: 0969-8051, DOI: 10.1016/0969-8051(95)02006-3 cited in the application

## Description

### Technical field of the invention

The present invention relates to a radiopharmaceutical solution comprising free/uncomplexed/un-chelated ²²⁴Ra and a complexing agent capable of scavenging/complexing/chelating ²¹²Pb and/or ²¹²Bi. This solution can be used for medical purposes, including treatment of cancer. Further aspects of the invention relates to kits and methods for providing specific solutions.

### Background of the invention

Targeted alpha particle radionuclide therapy holds promise as therapeutic modality against malignant and non-malignant diseases. Alpha-emitting radionuclides are highly cytotoxic and the alpha particles produced are of high linear energy, which is delivering a high amount of ionization over a short range, causing destruction of DNA by a high degree of irreparable double strand breaks.

Thus, it is important when using alpha emitting radionuclides that they do reach the target and are not released from the targeting compound and that they do not produce longer lived daughter nuclides that diffuse away from the mother nuclide, since this can cause toxicity in remote tissues.

There are relatively few alpha emitters considered for medical applications. It is a challenge in the field to find a radionuclide with appropriate half-life, decay properties, chemical properties and daughter products that are suitable for development of medical treatments.

Radium was very important for the development of radiochemistry sciences and was also used by the pioneers of radio-oncology to treat cancer with brachytherapy (, that is radiation emitting needles or seeds placed within or nearby tumors. Initially ²²⁶Ra with a half-life (t_{1/2}) of 1600 years was used. Later on ²²⁴Ra (t_{1/2} = 3.66 days), as dissolved radium chloride injectates, was used for several decades in Germany for the palliative treatment of ankylosing spondylitis (AS) because of the natural bone-seeking property of this, the heaviest of the alkaline earth elements.

Although reintroduced after the development of improved purification methods for a brief period about year 2000, its use was finally abandoned partly due to fear of late effects and the appearance of new treatment options for AS. Therefore, ²²⁴Ra is not in use as a radiopharmaceutical today and is not considered among the plausible candidates for alpha therapy by leading experts in the field.

There is, however, research going on using ²²⁴Ra loaded wires for local intratumoral brachytherapy/radon diffusion therapy but they are not using aqueous ²²⁴Ra solutions for therapy.

Recently, another radium isotope, ²²³Ra, in the form of a dissolved salt injectate, has been granted market authorization as a treatment against skeletal metastases from castration resistant prostate cancer.

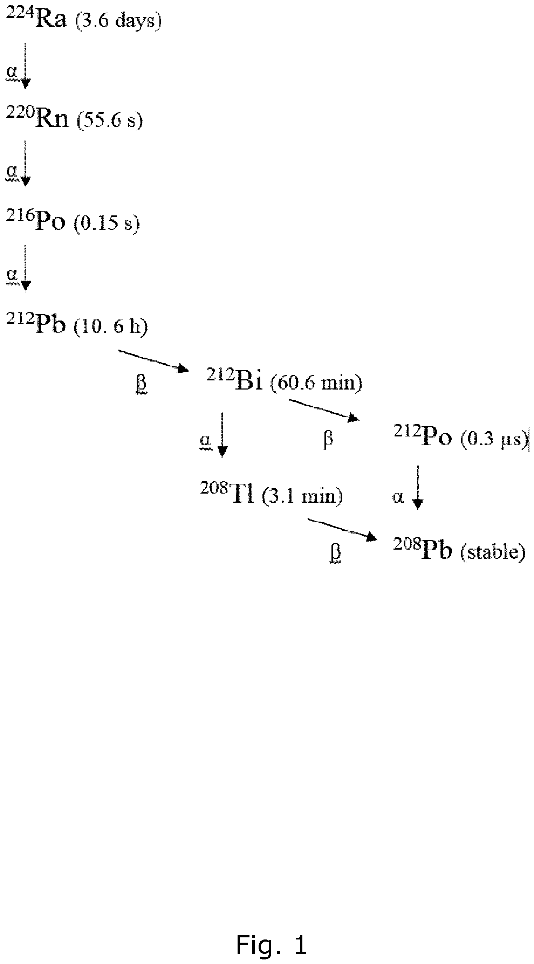

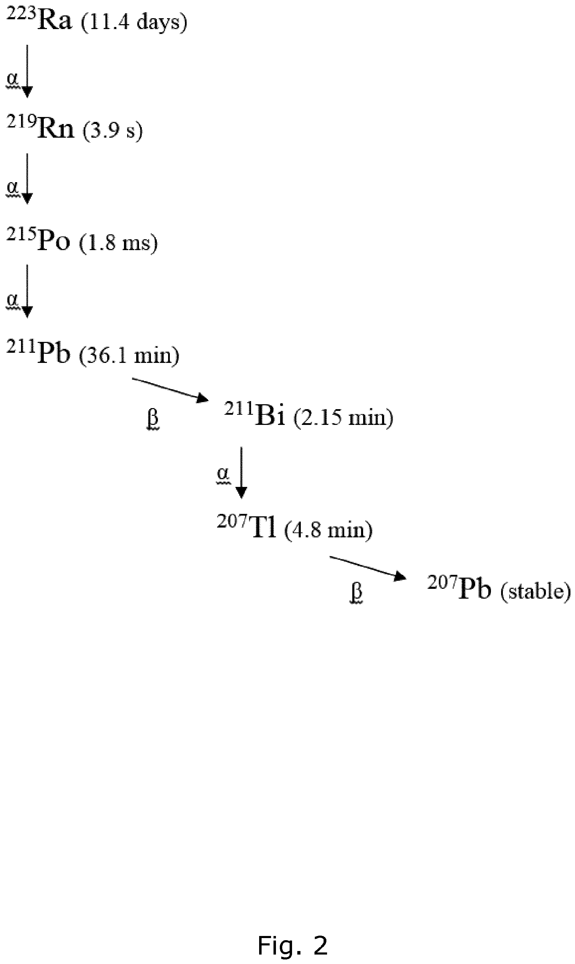

When comparing ²²³Ra (t_{1/2} = 11.4 days) with ²²⁴Ra (t_{1/2} = 3.6 days) both have, in principal, relevant half-lives for radiopharmaceutical uses allowing centralized production and shipment to the end user and both have three alpha emitting progenies in their decay chains and the series are producing a similar amount of alpha particles (Figures 1 and 2) with total alpha energy of about 26-28 MeV for the respective chains.

When comparing the decay chains, ²²³Ra progenies of the Rn, Pb and Bi elements have significant shorter half-lives reducing the problem of daughter nuclide uptake in non-target cells and tissues. These differences are particularly important for the lead progenies as these can accumulate in hematopoietic cells and tissues and in kidneys, respectively. In the ²²³Ra series ²¹¹Pb (t_{1/2} = 36.1 min) would cause much less normal tissue exposure compared with ²¹²Pb (t_{1/2} = 10.6 hours) from the ²²⁴Ra series. Unless ²²⁴Ra is purified from ²¹²Pb, short before injection, ²²³Ra would have significantly less normal tissue exposure from progenies. Such purification is impractical since it would require laborious procedures to be performed at the hospital where the product is being used or that the production and use is being geographically restricted. That is why the time frame for use of ²²⁴Ra that was previously supplied by Altmann Terapie, Salzgitter, Germany, for AS, was of only 6 hours. It could be used 3 hours before or three hours after the calibration time point. Probably to a significant extent because of this short product shelf life (in addition to increased competition with the new drugs for AS) and the thereby logistics and supply constraint the product has been discontinued.

As of currently, there is no use of ²²⁴Ra solution for injection to patients. Instead, there is in development ion exchanger based ²²⁴Ra generators for the extraction of ²¹²Pb for the use of ²¹²Pb in radioimmunotherapy. Lead-212 is itself a beta-emitter but decays to the alpha-emitter ²¹²Bi and is therefore considered suitable as an *in vivo* generator for alpha particle therapy.

Thus, currently ²²⁴Ra is considered merely as a generator nuclide for the medically useful ²¹²Pb. Because of the relatively short half-life of ²¹²Pb it is expected to be best suited in treatment against compartmental disease where the radioimmunoconjugate is injected directly into the region, e.g., intraperitoneal (*i.p*.) cavity where a high concentration of product may target malignant ascites and micrometastases within the cavity. The half-life of 10.6 hours of ²¹²Pb may be of benefit as only low amount leaks out from the *i.p.* cavity before the radioactivity has decayed.

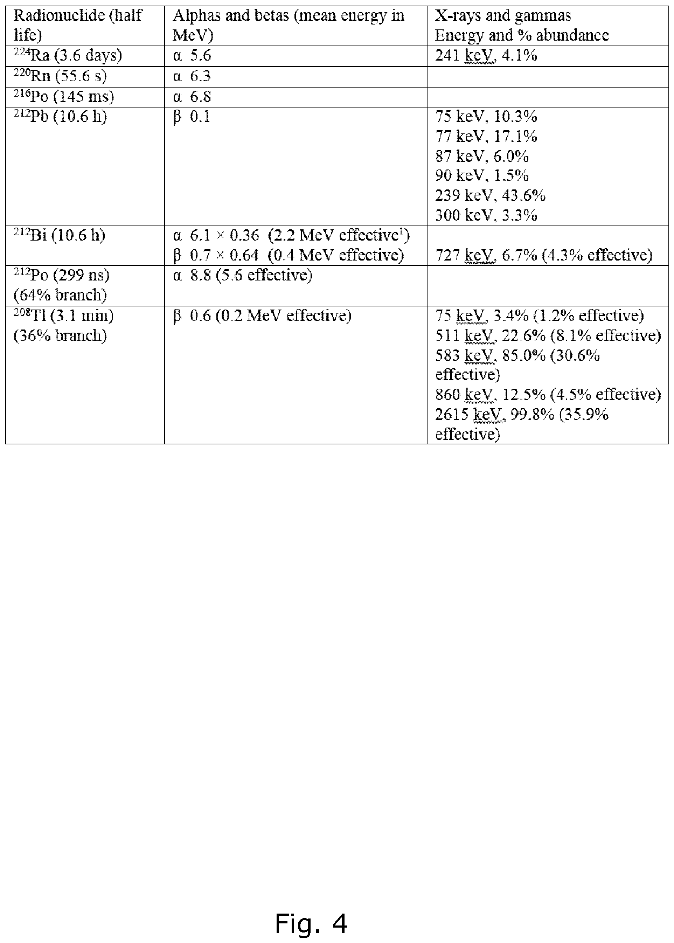

When considering radium for therapy against bone diseases, it should be kept in a cationic state since this will ensure that that radium, as the so-called "volume-seeker" it is, will be built into the bone minerals causing retention of the daughter nuclides. This is particularly important with ²²⁴Ra since some of the daughter nuclides, in particular ²¹²Pb, have substantial half-lives allowing trans-organ redistribution if let free in physiological liquids like blood, saliva or lymphatic liquid. Figure 4 lists the main radiation of the decay chain of ²²⁴Ra.

A monograph about the use of ²²⁴Ra in ankylosing spondylitis were developed by the German health authorities about 10 years ago when Altmann Terapie (Salzgitter, Germany) made an effort to re-introduce ²²⁴Ra solution as a treatment against ankylosing spondylitis based on a patented production method yielding a highly purified product.

In the decay chain of ²²⁴Ra the daughter product ²¹²Pb (t_{1/2}= 10.6 hours) is produced. It has a different bio distribution compared to the ²²⁴Ra mother nuclide when co-injected in patients. This causes less initial activity in the target tissues and more activity in the non-target tissues like blood cells, in particular hematopoietic cells and tissues, and bone marrow and kidneys. The number of ²¹²Pb atoms compared with ²²⁴Ra in a product in radioactive equilibrium is less than 14%. But because ²²⁴Ra rapidly transfer from blood to the skeleton or is excreted and ²¹²Pb is substantially retained in hematopoietic cells and tissues, the toxicological impact of ²¹²Pb, which generates the alpha emitter ²¹²Bi, is important. The only way to solve this problem by current knowledge in the field would be to use ²²⁴Ra short time after purification as suggested, that is, before a significant in-growth of ²¹²Pb has taken place.

The use of scavenger for daughter products in experimental radiopharmaceutical research has been described: Jones *et al* (1996) studied oral administration over several days of the non-targeted dithiol chelating agents 2,3-dimercapto-1-propanesulfonic acid (DMPS) and meso-2,3-dimercaptosuccinic acid (DMSA) to improve the clearance of ²⁰⁶Bi from kidneys in mice and found improved kidney clearance with DMPS. Their aim was to use oral chelate as potential adjuvants to reduce or prevent radiotoxicity in anti-interleukin-2 receptor (IL-2R) ²¹²Pb or ²¹²Bi alpha-radioimmunotherapy. Jaggi *et al.,* (2005) used oral chelation therapy to reduce renal accumulation of ²¹³Bi produced from ²²⁵Ac. Their goal was to increase excretion of the undesired daughter product. They did not add the chelator to the radiopharmaceutical but merely describe its use as oral medication in the drinking water before and after injection of radiopharmaceutical. Piotrowska *et al.,* (2013) discloses the use of Na nanozeolites as carriers for ²²⁴Ra and ²²⁵Ra targeted radionuclide therapy.

In terms of bone therapy, ²²³Ra is considered more appropriate than ²²⁴Ra because ²²³Ra have daughter nuclides with much shorter half-lives and thus, less problem of relocalization. Radium-223 has recently been approved for the therapy of patients with hormone refractory skeletal metastases from prostate cancer.

Dissolved ²²⁴Ra salt has previously been tested in cancer therapy but was abandoned because of unfavorable properties and ineffectiveness. It was stated that because of the short half-life of ²²⁴Ra and its injected daughters, the soft tissue(s) are irradiated. In other words, in the case of ²²⁴Ra the half-life of the daughters, in particular ²¹²Pb, are relatively long, compared to the mother nuclides and more soft tissue exposure occurs. Therefore, it is known in the field that ²²⁴Ra has unfavorable daughter nuclide restricting its use in radiopharmaceutical solutions. Also, in recent review by senior experts in the field, ²²⁴Ra was not listed among the candidate radionuclides considered for alpha particle emitter radiopharmaceutical therapy.

The concept of circulating tumor cells (CTC) has lately received considerable attention as CTC may play a critical role in the development of tumor metastases. It is known that cancers that produce skeletal metastases like e.g., prostate-, breast-, lung- and multiple myeloma cancers may have viable circulating cancer cells in the blood, which may have been shed from the primary or metastatic tumors. This means that even if the bone metastases are treated new lesions can be formed by the settlement of CTC's in the bone or other tissues.

Radium-223 used against bone metastases today is a pure bone-seeker and do not address the problem of CTC's. Therefore, there is a need in the field of alpha pharmaceutical bone therapeutics of a product that can also address CTC's.

The generation of daughter nuclides both in the injectates and *in vivo* is a potential problem for ²²⁴Ra and to a lesser extent ²²³Ra as the first progeny in the two decay series for both is radon which is highly diffusive. However, literature data indicate that this is less of a problem when generated *in vivo* since radium is a bone volume-seeker and is embedded in the bone matrix. It also helps out that the uptake in skeleton of intravenous radium occurs almost instantly and intestinal, and to a less degree urinary, elimination happens rapidly, leading to an elimination from the blood within minutes after injection. It should be mentioned that urinary elimination is probably more pronounced in rodents compared to humans were fecal elimination is the main route. As reported by Nilsson *et al* (2005), a reduction of 88% of radium in blood at 10 minutes after injection occurs. When considering radium localized in the skeleton, it was for ²²³Ra reported equilibrium of ²¹¹Bi and ²²³Ra in bone after a few hours. For ²²⁴Ra based on animal data and extrapolation to adult human, it was found by two different models a ²¹²Pb to ²²⁴Ra fraction of 0.88 and 1.0, *i.e.,* almost complete retention of daughters. These data indicates a high retention of the daughter nuclides in bone for both ²²³Ra and ²²⁴Ra. For ²²⁴Ra a significant contribution to soft tissue uptake of progeny would therefore likely be from co-injected daughter nuclide. Thus it is imperative to develop methods to control daughter nuclides, at least ²¹²Pb, in ²²⁴Ra injectates.

This has been achieved by the new ²²⁴Ra solutions described herein.

### Summary of the invention

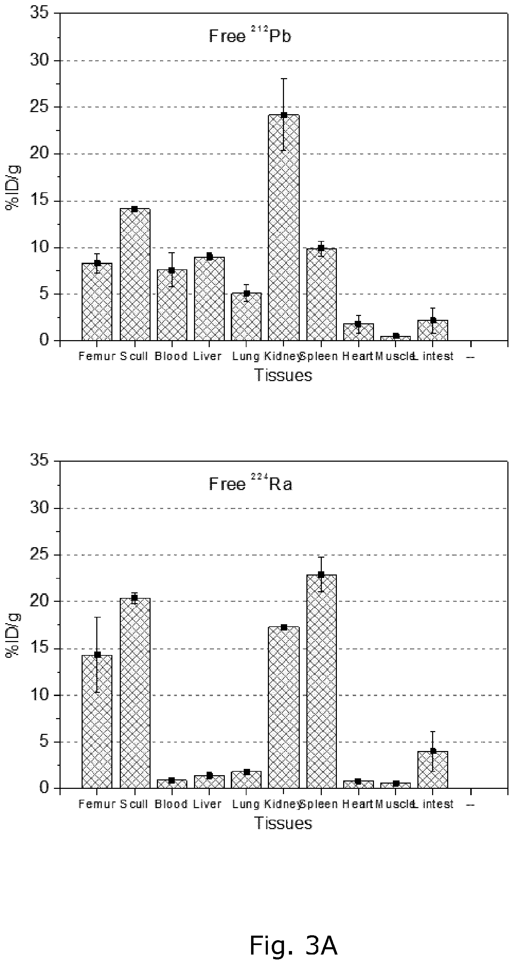

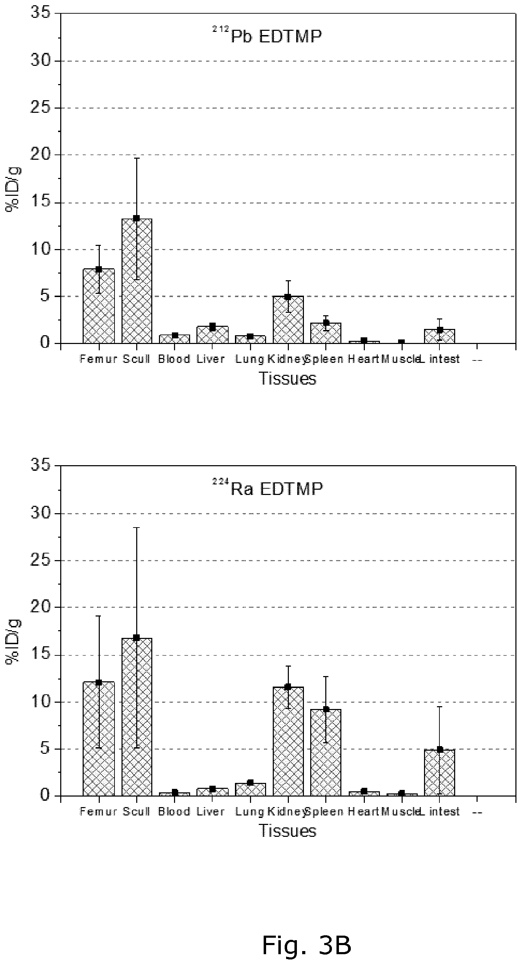

The present invention relates to radiopharmaceutical compositions comprising the mother nuclide ²²⁴Ra, its daughter nuclide ²¹²Pb, and a complexing agent that complexes with the daughter nuclide. While ²²⁴Ra targets the bone in its un-complexed form, an embodiment of the composition comprises the complexing agent EDTMP which complexes with ²¹²Pb and specifically targets it to the bone, thus avoiding and/or minimizing undesirable side effects and/or off-target effects due to ²¹²Pb. This is evident in a comparison of e.g. Fig. 3A and Fig. 3B. In another embodiment, the complexing agent is TCMC-labeled monoclonal antibody, e.g., trastuzumab (Herceptin) which enables specific targeting of ²¹²Pb to the blood. This is an unexpected feature very useful in a situation where, for example, there are circulating tumor cells in the blood. Thus, therapeutic efficacy above and beyond that available with ²²⁴Ra can e.g. be achieved in a target-specific manner by specifically controlling ²¹²Pb targeting by the bone-seeking EDTMP and blood-circulating TCMC-labeled monoclonal antibody. Mice *in vivo* studies are presented in example 12, showing an improvement compared to the gold-standard treatment protocol.

An object of the present invention is to provide a radiopharmaceutical solution comprising ²²⁴Ra and a complex capable of scavenging at least ²¹²Pb.

In one embodiment of the present invention, the complex comprises one or more compounds selected from the group consisting of acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, TCMC, DOTA, p-SCN-Bn-DOTA, p-SCN-Bn-TCMC, antibody-conjugated-DOTA, and antibody-conjugated-TCMC.

In another embodiment of the present invention, ²¹²Pb and/or ²¹²Bi is complexed by (bone-seeking) EDTMP.

In another embodiment of the present invention, the complexing agent is conjugated to a compound selected from the group consisting of a monoclonal, a polyclonal antibody, an antibody fragment, a synthetic protein, peptide, vitamin or vitamin derivative.

In another embodiment of the present invention, the complexing agent is the chelator TCMC conjugated to a compound selected from the group consisting of a monoclonal, a polyclonal antibody, am antibody fragment, a synthetic protein, peptide, vitamin or vitamin derivative.

One aspect of the present invention relates to a kit comprising a first vial comprising a radiopharmaceutical solution of the present invention, and a second vial comprising a neutralizing solution to adjust pH and/or isotonicity of the radiopharmaceutical solution prior to administration to a patient.

Another aspect of the present invention relates to a kit comprising a first vial comprising a chelate labelled protein or peptide, a second vial comprising a ²²⁴Ra solution.

A further aspect of the present invention relates to a radiopharmaceutical solution of the present invention for use as a medicament.

Yet another aspect of the present invention relates to a radiopharmaceutical solution of the present invention for use in treating skeletal disease.

In one embodiment of the present invention, the skeletal disease is selected from the group consisting of skeletal metastases from cancers to the breast, prostate, kidneys, lung, bone, or multiple myeloma, or non-cancerous diseases causing undesired calcification including ankylosing spondylitis.

Another aspect of the present invention relates to a method for providing a radiopharmaceutical solution according to claim 15.

### Brief description of the figures

Figure 1 shows the decay of ²²⁴Ra and daughters.
Figure 2 shows the decay of ²²³Ra and daughters.
Figure 3A shows the bio-distribution in nude mice of ²²⁴Ra and progeny ²¹²Pb in a solution without chelator. Figure 3B shows bio-distribution in nude mice of ²²⁴Ra solution containing EDTMP as chelator for progeny ²¹²Pb. Figure 3C shows biodistribution in nude mice of ²²⁴Ra solution containing TCMC-trastuzumab (Herceptin) as chelator for progeny ²¹²Pb.
Figure 4 shows main radiation properties from the ²²⁴Ra series. ¹Average per ²²⁴Ra transformation due to branching. Only X-rays or gammas above 1% effective abundance are accounted for. It adds up to a total effective energy of approximately 26.5 MeV of alpha of 0.7 MeV of beta per complete decay of ²²⁴Ra and daughters.
Figure 5 shows ingrowth of ²¹²Pb from a pure 10 MBq ²²⁴Ra source. Changes in activity level. Start activity 10 MBq pure 224-Ra.
Figure 6A shows thin layer chromatographic (TLC) profiles of ²¹²Pb in a solution of ²²⁴Ra in equilibrium with progeny nuclides without and with complexing agents EDTMP and DOTMP.
Figure 6B shows thin layer chromatographic (TLC) profiles of ²¹²Pb in a solution of ²²⁴Ra in equilibrium with progeny nuclides without and with complexing agents.

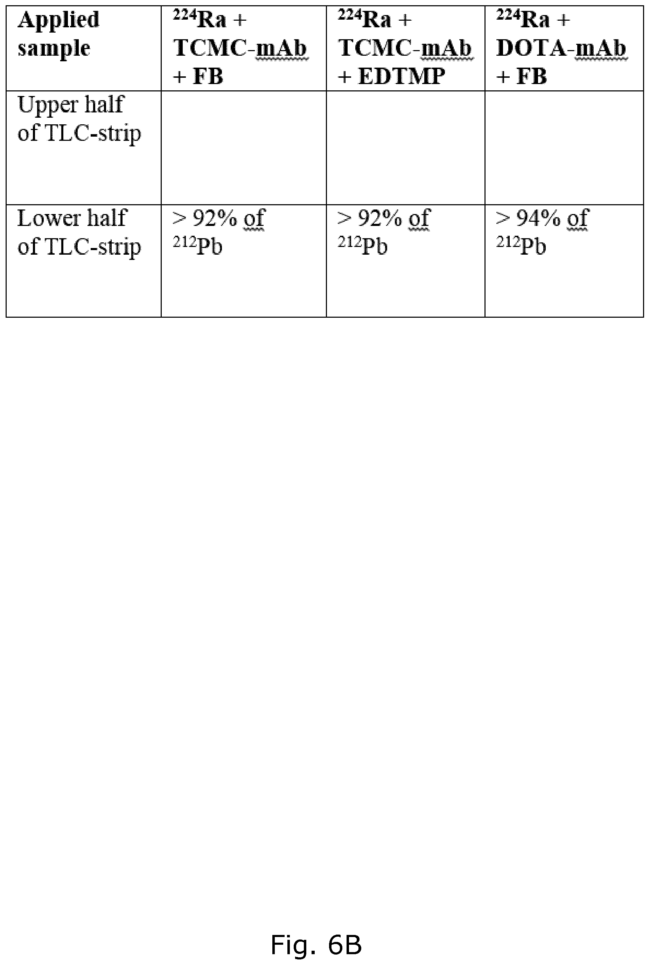

Figure 6B shows TLC profiles for ²¹²Pb in the presence of antibody- TCMC or DOTA conjugates and antibody without chelator.

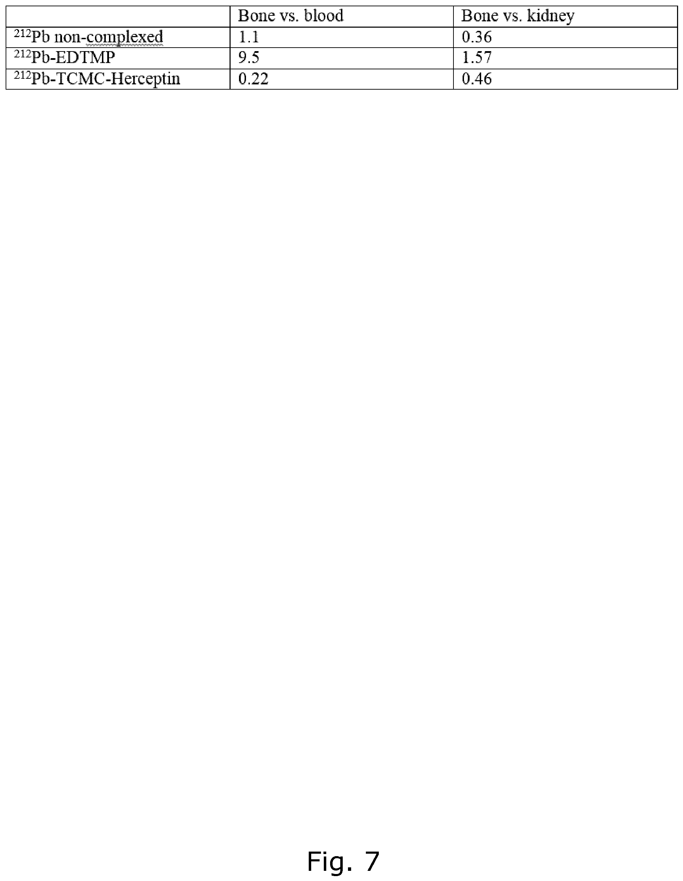

Figure 7 shows uptake ratios* for ²¹²Pb for bone vs. blood and bone vs. kidneys.

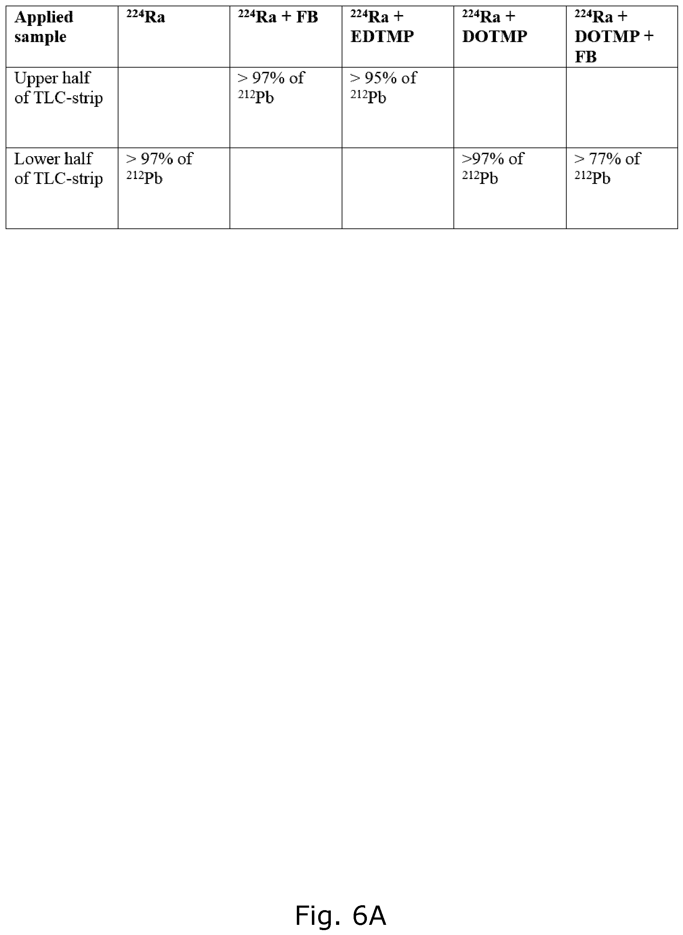

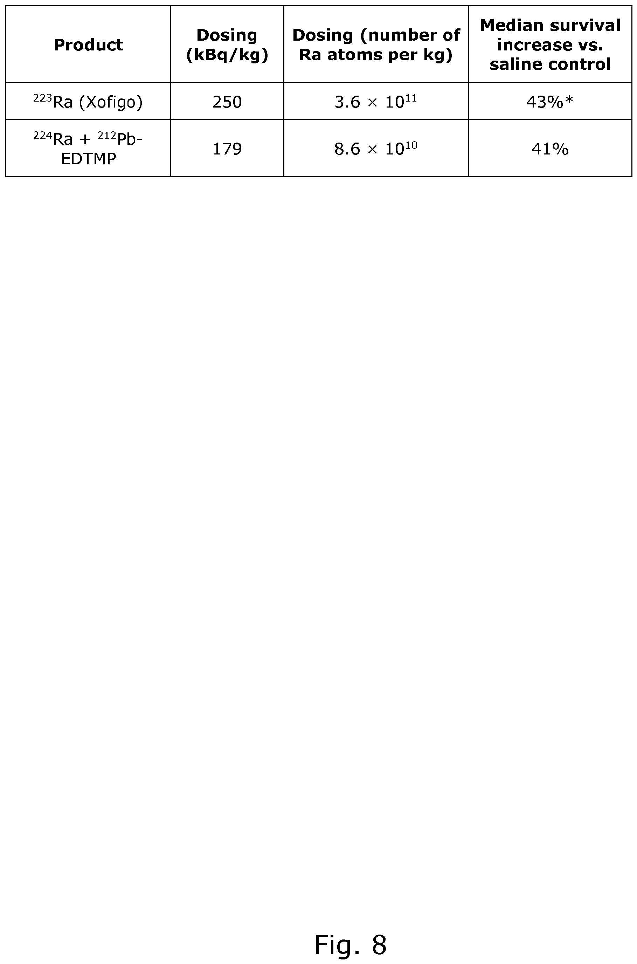

Figure 8 shows comparison of data for ²²³Ra and ²²⁴Ra + ²¹²Pb-EDTMP in the MDA-MB-231 (SA) model in nude mice. *From Suominen et al., J Natl Cancer Inst, 2013, 105: 908-916, Figure 6, p. 915.

The present invention will now be described in more detail in the following.

### Detailed description of the invention

Some abbreviations used
DOTMP - 1,4,7,10-tetraazacyclododecane-1,4,7,10-tetra(methylene phosphonic acid)
EDTMP - ethylenediamine tetra(methylene phosphonic acid)
EDTA - ethylenediaminetetraacetic acid
p-SCN-Bn-DOTA - 2-(4-isothiocyanatobenzyl)-1,4,7,10-tetraazacyclododecane-1,4,7,10-tetraacetic acid
DOTA- 1,4,7,10-tetraazacyclododecane-1,4,7,10-tetraacetic acid and also used for benzyl-1,4,7,10-tetraazacyclododecane-1,4,7,10-tetraacetic acid (e.g. conjugated to monoclonal antibody)
p-SCN-Bn-TCMC - 2-(4-isothiocyanotobenzyl)-1, 4, 7, 10-tetraaza-1, 4, 7, 10-tetra-(2-carbamonyl methyl)-cyclododecane
TCMC - 1, 4, 7, 10-tetraaza-1, 4, 7, 10-tetra-(2-carbamonyl methyl)-cyclododecane and also used for benzyl-1, 4, 7, 10-tetraaza-1, 4, 7, 10-tetra-(2-carbamonyl methyl)-cyclododecane (e.g. conjugated to monoclonal antibody) mAb -monoclonal antibody.

The same abbreviations are in the following used for acids, salts or partly or fully dissociated versions of the chelators.

It has been an unexpected finding that it is possible to strongly complex daughter nuclide in the presence of radium with complexing compounds tested, EDTMP and monoclonal antibody conjugated to the TCMC and DOTA chelators, and at the same time keeping radium (²²⁴Ra) mainly as un-complexed cation fully targetable to bone.

### A radiopharmaceutical solution

An object of the present invention is to provide a radiopharmaceutical solution comprising ²²⁴Ra and a complex capable of scavenging at least ²¹²Pb.

Thus, an aspect of the invention relates to a radiopharmaceutical solution comprising un-complexed ²²⁴Ra and complexes between a complexing agent and ²¹²Pb. Preferably the complexing agent is selected from the group consisting acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, and antibody-conjugated-TCMC. Even more preferably, the complexing agent is a cyclic chelator or an acyclic chelator.

It is to be understood that the complexing agent according to the invention may also cover derivatives of the above-mentioned compounds (such as derivatives of EDTMP, DOTA and TCMC). It of course to be understood that such derivatives must maintain the capability to complex ²¹²Pb with a higher stability constant than to ²²⁴Ra. Thus in an alternative embodiment, the complexing agent is selected from the group consisting acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, antibody-conjugated-TCMC or derivatives of any of these; wherein said derivatives complex ²¹²Pb with a higher stability constant than to ²²⁴Ra.

Suitable chelators include DOTA derivatives such as p-isothiocyanatobenzyl-1,4,7,10-tetraazacyclododecane-1,4,7,10-tetraacetic acid (p-SCN-Bz-DOTA) and DOTA-NHS-ester.

Another aspect of the invention relates to a radiopharmaceutical solution comprising ²²⁴Ra, ²¹²Pb and a complexing agent selected from the group consisting of EDTMP, antibody-conjugated-DOTA, or antibody-conjugated-TCMC.

In an embodiment, the complexing agent is capable of complexing at least ²¹²Pb.

In another embodiment, the complexing agent is capable of complexing a daughter nuclide of ²²⁴Ra, such as ²¹²Pb, in the pharmaceutical solution.

The complexing agent does not complex ²²⁴Ra or substantially complex ²²⁴Ra in the pharmaceutical solution.

In yet a further embodiment, the complexing agent complexes with a higher stability constant to ²¹²Pb than to ²²⁴Ra.

In an embodiment, the stability constant for ²¹²Pb is at least twice the affinity for ²²⁴Ra, such as at least four times higher, such as at least 8 times higher or such as at least 10 times higher.

In another embodiment, the complexing agent does not affect or substantially affect the bio-distribution of ²²⁴Ra in vivo.

In Example 6 (figures 3A vs 3B) it can be seen that when EDTMP is used as complexing/chelating agent, ²¹²Pb is redistributed primarily to the bones, whereas the distribution of ²²⁴Ra is almost not affected

In Example 9 (figures 3A vs 3C) it can be seen that when TCMC-Herceptin is used as complexing/chelating agent, ²¹²Pb is redistributed primarily to the blood, whereas the distribution of ²²⁴Ra is almost not affected.

In yet an embodiment, the complexing agent is selected from the group consisting of acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, and antibody-conjugated-TCMC,
wherein the complexing agent is capable of complexing a daughter nuclide of ²²⁴Ra, such as ²¹²Pb, in the pharmaceutical solution, and wherein the complexing agent does not complex ²²⁴Ra in the pharmaceutical solution.

In a preferred embodiment said complexing agent is selected from the group consisting of EDTMP, antibody-conjugated-DOTA, or antibody-conjugated-TCMC.

In a further embodiment, the amount of ²²⁴Ra and ²¹²Pb is in radioactive equilibrium.

In yet a further embodiment, the activity ratio (in MBq) between ²¹²Pb to ²²⁴Ra is between 0.5 and 2, such as 0.8 - 1.5, or such as 0.8 - 1.3, or preferably such as 0.9 - 1.15.

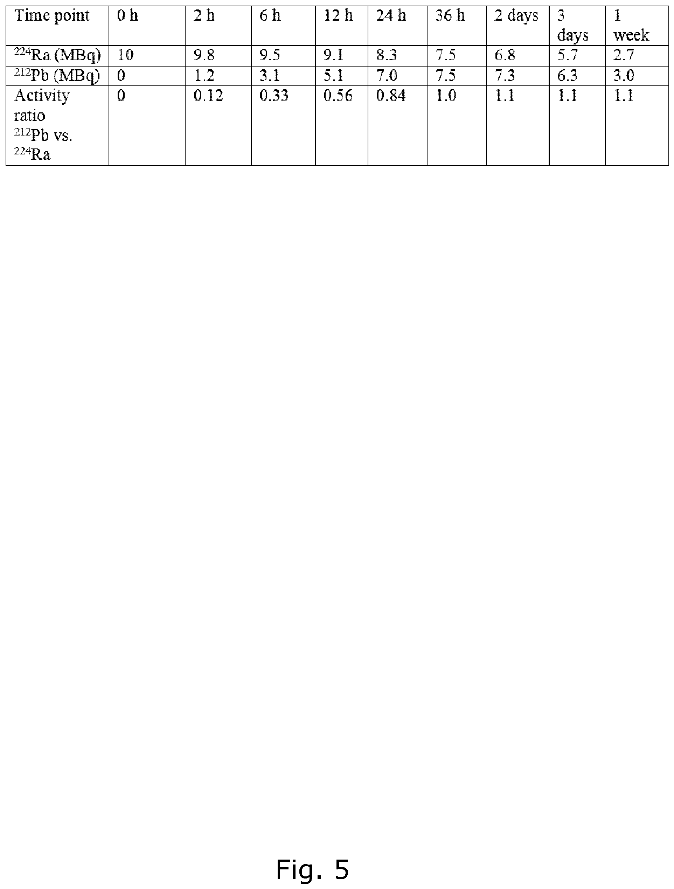

In the present context, the term "radioactive equilibrium" relates to the ratio in MBq between two radionuclides being the same or substantially the same over time. The term "activity ratio" e.g. between ²¹²Pb and ²²⁴Ra relates to the ratio of MBq of ²¹²Pb to ²²⁴Ra. In figure 5 is a table (table 2) showing the development of this activity ratio over time. It can be seen that after two days a radioactive equilibrium of 1.1 has been established for the activity ratio between ²¹²Pb to ²²⁴Ra (7.3 divided by 6.8). Thus, in figure 5, it can also be seen that the radioactive equilibrium between ²¹²Pb and ²²⁴Ra is reached after about 2 days.

In the present context, the terms "complexing agent", "scavenger" and "chelating agent" are used interchangeably. The terms relate to agents capable of forming complexes with ²¹²Pb, preferably by chelation and with a significant strength as measured in test systems while radium is not significantly affected by the presence of the complex as measured in the test systems. Test systems: *In vivo* biodistribution and *in vitro* cation exchanger or size retention and centrifuge concentration cartridge for chelate-antibody binding of radionuclide.
In the present context "scavenging" (or complexing) is defined as at least 50% bound according to thin layer chromatography (TLC), centrifuge concentration separation or bio-distribution profiles.

This means, as an example, at least 50% less blood uptake of ²¹²Pb with a small molecular chelator. With an antibody-conjugated chelator, where blood uptake is not a reliable indicator, at least 50% bound according to TLC analyses.

In one embodiment of the present invention is at least 60% bound.

In another embodiment of the present invention is at least 70% bound.

In another embodiment of the present invention is at least 80% bound.

In another embodiment of the present invention is at least 85% bound.

In another embodiment of the present invention is at least 90% bound.
The compound or compounds may also be capable of scavenging more radionuclides than ²¹²Pb.

In one embodiment of the present invention, the complex comprises one or more compounds selected from the group consisting of acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, and antibody-conjugated-TCMC.

In one embodiment of the present invention, the complex is at a concentration of 1 ng/mL to 1g/mL.

In another embodiment of the present invention, the complex is at a concentration of 100 ng to 10 mg/mL
The complex can comprise one, two, three, four, five or more compounds.

In another embodiment of the present invention, ²¹²Pb and/or ²¹²Bi is complexed by bone-seeking EDTMP.

In one embodiment is the solution in a volume of 100 µL to 1000 mL, such as 500 µL to 100 mL, 1 mL to 10 mL.

In one embodiment of the present invention is the radioactivity of the solution 1 kBq to 1 GBq, such as 10 kBq to 100 MBq, such as 100 kBq to 10 MBq.

In another embodiment of the present invention is the radioactivity of the solution 100 kBq to 100 MBq.

In another embodiment of the present invention, the complexing agent is conjugated to a compound selected from the group consisting of a monoclonal, a polyclonal antibody, an antibody fragment, a synthetic protein, a peptide, a hormone or hormone derivative or a vitamin or vitamin derivative, *e.g.,* biotin and folate.

In another embodiment of the present invention, the complexing agent is the chelator TCMC conjugated to a compound selected from the group consisting of a monoclonal, a polyclonal antibody, an antibody fragment, a synthetic protein, a peptide, a hormone or hormone derivative or a vitamin or a vitamin derivative.

For dosing purposes ²²⁴Ra solutions administered should have been stored for some time, *e.g.,* 1 day or more preferably at least two days, such as 1-2 days or 1-3 days, to reach equilibrium between ²²⁴Ra and ²¹²Pb/²¹²Bi. This will ensure ²¹²Pb to ²²⁴Ra activity ratios between 0.83 and 1.14. This can, *e.g.,* be accomplished by the manufacturer by simply retaining the product for a day or so before shipment.

It is important to keep radium as mainly un-complexed, or weakly complexed cation as this ensures a maximum uptake in bone and bone metastases and also ensures favourable excretion of eliminated product mainly through the intestines.

By adding complexing agent to a solution of radium the radioactive daughter can be made bone- or tumor-seeking and increase the therapeutic potential of the radium solution instead of being a health hazard. It should be a complexing agent that does not negatively affect the bone-seeking properties of radium, though. For example can EDTMP scavenge ²¹²Pb produced in the radium solution during transport and storage between the production site and the hospitals whereby the product is going to be administered.

Although it is possible to reduce the susceptibility by adding radiolytic inhibitors, tumor targeting proteins or peptides are often more susceptible to radiolysis and should probably be supplied in a kit format whereby they are added a few hours to a few minutes before administration of ²²⁴Ra solutions with relatively long shelf-lives.

It is known in the field that calixarenes and EDTA to some extent can complex radium and also complex lead and bismuth. However, in the current work we found chelators that would leave radium mainly uncomplexed or weakly complexed, as determined by *in vivo* biodistribution measurements, while being able to rapidly and with relevant stability, complex the longest living daughter ²¹²Pb. The selective complexation can be used to make at least lead bone- or tumor-seeking while maintaining the favourable properties of radium in terms of treating sclerotic diseases, like skeletal metastases. The ²¹²Pb complex that targets bone or tumor cells generates the alpha emitter ²¹²Bi from the decay of ²¹²Pb. Thus, the beta emitter ²¹²Pb is used as an indirect alpha source for irradiating the targeted cells or tissue. Other potential chelates which could be suitable for ²²⁴Ra daughter nuclide scavenging besides TCMC and DOTA includes but are not limited to phorphyrins, DTPA and DTPA derivatives and also carboxyl linked DOTA.

Lead-212 is by far the longest living of the progenies from ²²⁴Ra and this is the most important to complex, as it is an *in vivo* generator for the short-lived alpha-emitter ²¹²Bi. If a ²¹²Pb-chelate is taken up in bone or in tumor cells ²¹²Bi will also likely be retained in the target. In a ²²⁴Ra solution in equilibrium with progenies there will be more than 10 times of ²¹²Pb vs.²¹²Bi atoms. Thus, the amount of radiation generated from the ²¹²Bi atoms in these solutions are modest and probably not of a toxicologically importance compared with ²²⁴Ra and ²¹²Bi decay series. The amount of ²¹²Bi is comparable to that of the ²¹¹Pb which indirectly produces an alpha particle in the ²²³Ra series and this has not been of a significant problem for the registration and clinical use of ²²³Ra in equilibrium with progenies.

If, however, a high degree of chelation also of ²¹²Bi in an injectate should be needed, it may at least in some instances be necessary to add a stabilizing agent like NaI or HI since bismuth in aqueous solutions tends to exist in a state less suitable for chelation.

When comparing with current approved alpha-pharmaceutical for treatment of skeletal metastases, *i.e.,* ²²³Ra, the novel solutions described herein could give, in one of the embodiment, a product with improved properties for treatment of skeletal metastases since the daughter nuclide can be made targetable to circulating cancer cells and, to some extent, also soft tissue metastases. This may prevent recurrence from cancer recolonization of the skeleton due to CTC's.

Another aspect is that the shorter half-life of ²²⁴Ra vs. ²²³Ra may actually be of some benefit as the radium is embedded in the bone matrix. Because of the high density of the bone mineral the range of alpha-particles is strongly reduced in bone vs. soft tissues. Especially in rapid mineralizing areas like osseous cancer metastases, the embedment process may be of significance when using a volume-seeking alpha-pharmaceutical.

Therefore, ²²⁴Ra could improve the tumor dose since, on average, it will be less embedded at the time of decay.

Diseases by which the novel ²²⁴Ra solutions may be used include primary and metastatic cancers, autoimmune diseases and artherioschlerosis. The product may be administered intravenously or locally, including intraperitoneally, or in limb perfusion settings.

The chelators used in the novel solutions may be acyclic as well as cyclic chelators and cryptands, crown ethers, porphyrins or cyclic- or noncyclic polyphosphonates including DOTMP and EDTMP. Also a bisphosphonate, *e.g.,* pamidronate, conjugated to DOTA, TCMC or similar may be used as scavenger in the ²²⁴Ra solution.

One may argue that the amount of ²¹²Pb in therapeutic ²²⁴Ra solution may be moderate to modest (*i.e.,* at equilibrium about 1.1 times that of ²²⁴Ra). If one assume similar dosing of ²²⁴Ra as is done with ²²³Ra in patients but correct for the half-life difference, roughly 150 kBq per kg of bodyweight would be the administered dose.

At equilibrium this would translate into a ²¹²Pb-antibody conjugate dosage of 11.5 MBq in 5 litres of blood in a 70 kg patient (if ²¹²Pb is quantitatively chelated). The number of circulating tumor cells is typically less than 10 cells per ml, thus in 5 l blood there are less than 50 000 tumor cell in total. If only 1 in 100 000 of the injected ²¹²Pb-antibody conjugate molecules binds to the tumor cells this would mean at least 0.0023 Bq per cell, equivalent to approximately 127 ²¹²Pb atoms bound per cell, which would be highly destructive as it has been reported that a mean of 25 cell bound ²¹²Pb per cell would kill 90% of a cell population.

Previously it was suggested to combine radium and phosphonates and more preferentially bisphosphonates in the treatment of skeletal metastases of cancer. However, it was suggested to use the two compounds separated from each other as it was preferred to inject at different time points. The application of phosphonates was not indicated for radionuclide complexation. The main purpose was to use pharmacologically active amounts of phosphonates as a secondary bone treatment to radium. Also it was preferred to use a non-complexing bisphosphonates, thus this teaches away from using EDTMP or similar as additive to radium solutions for the complexation of daughter nuclides. At the time it was common knowledge that EDTMP could complex alkaline earth metals, as it was known in the field that ¹⁵³Sm-EDTMP can cause complexing of calcium in blood and cause hypocalcemia.

However, in the current work we have shown that when modest amounts of EDTMP is used, it is possible to complex ²¹²Bi and ²¹²Pb without significantly reducing the bone-seeking properties of ²²⁴Ra.

The current report is the first time the addition of a complexing phosphonate to a radium solution has been presented. It shows that it is possible to obtain selective complexation of daughter nuclide without significantly alter the bone targeting properties of radium. This is important since although complexing phosphonates are bone-seekers, radium shows an even higher bone targeting ability than the phosphonates. It is therefore highly advantageous that the labeling of daughter nuclides does not cause reduced skeletal uptake of the radium.

As for bone targeting with phosphonates it is known in the field that radionuclides like ¹⁷⁷Lu, ¹⁵³Sm, ²²⁷Th, and ²²⁵Ac complexed with phosphonates can target bone. In a previous report, it was also shown that the radionuclides ²¹²Pb and ²¹²Bi could be complexed with EDTMP and DOTMP. However, the labelling was performed with high pH and also needed to be purified by ion exchanger after labelling. Thus, this teaches away from using *in situ* labelling in the presence of radium, without affecting radium, and without purification as is shown in the current application. Thus, we hereby present a novel way of using EDTMP as a scavenger for daughter nuclides in ²²⁴Ra solutions which (1) improve the bone dose per unit ²²⁴Ra administered, and (2) strongly reduce uptake of ²¹²Pb in hematopoietic cells and tissues in effect causing better target to non-target radiation ratios.

The solutions used for ²²⁴Ra and the daughter nuclide complexation may contain radiolytic inhibitors and other modificators suitable for a medical injectate known in the field.

The solution can also be a pharmaceutical composition.

Usually is an important element of a pharmaceutical composition a buffer solution, which to a substantial degree maintain the chemical integrity of the radioimmunoconjugate and is being physiologically acceptable for infusion into patients.

In one embodiment of the present invention, the pharmaceutical composition comprises one or more pharmaceutically acceptable carriers and/or adjuvants.

Acceptable pharmaceutical carriers include but are not limited to non-toxic buffers, fillers, isotonic solutions, etc. More specifically, the pharmaceutical carrier can be but are not limited to normal saline (0.9 %), half-normal saline, Ringer's lactate, 5 % Dextrose, 3.3 % Dextrose/0.3 % Saline. The physiologically acceptable carrier can contain an anti-radiolytic stabilizer, e.g., ascorbic acid, which protect the integrity of the radiopharmaceutical during storage and shipment.

### Kits

The solution should be made physiologically suitable for injections either at a centralized production site or be made up by a kit system of typically 2-4 vials whereby being physiologically suitable for injection after combination of the kit vials.

One aspect of the present invention relates to a kit comprising a first vial comprising a radiopharmaceutical solution of the present invention, and a second vial comprising a neutralizing solution to adjust pH and/or isotonicity of the radiopharmaceutical solution prior to administration to a patient.
Another aspect of the present invention relates to a kit comprising a first vial comprising a chelate (complexing agent) conjugated to a protein or peptide or mixtures of proteins or peptides, and a second vial comprising a ²²⁴Ra solution.

Since the decay series of ²²⁴Ra includes a radon daughter, which may diffuse into the air, vials containing the products must be well sealed to prevent escape of ²²⁰Rn.

Because of the highly localized nature of alpha-irradiation, radiolysis must be considered as a potential problem and the radiopharmaceutical must be designed to minimize this. According to the knowledge in the field, radiolabeled antibodies are sensitive to radiolysis and therefore a kit system may be advantageous for ²²⁴Ra solutions, which are to be combined with chelator conjugated antibodies for scavenging ²¹²Pb and or ²¹²Bi.

For a monoclonal antibody it is usually advisable be keep the self-dose of the alpha particle producing radiopharmaceutical solution below 0.5 kGy to avoid reduced binding properties due to radiolysis. Thus, a kit system whereby chelator conjugated antibody is added to the ²²⁴Ra (including daughters) solution a few hours to 10 minutes before injection is advised for concentrated solutions intended for remote shipping.

A further aspect of the invention relates to a kit comprising
- a first vial comprising a radiopharmaceutical solution according to the invention, and
- a second vial comprising a neutralizing solution to adjust pH and/or isotonicity of the radiopharmaceutical solution prior to administration to a patient.

Yet an aspect relates to a kit comprising
- a first vial comprising a ²²⁴Ra solution;
- a second vial comprising a complexing agent selected from the group consisting of acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, and antibody-conjugated-TCMC, wherein the complexing agent is capable of complexing a daughter nuclide of ²²⁴Ra, such as ²¹²Pb, and wherein the complexing agent does not (or substantially not) complex ²²⁴Ra in the pharmaceutical solution; and
- optionally, instructions for mixing the first vial and the second vial, thereby forming a pharmaceutical composition ready to be administered to a patient 1 minute to 12 hours after mixing.

In a preferred embodiment said complexing agent is selected from the group consisting of EDTMP, antibody-conjugated-DOTA, or antibody-conjugated-TCMC.

In a specific embodiment, the term "²²⁴Ra solution" is to be understood as ²²⁴Ra is free in the solution and not coupled to e.g. a surface such as a resin.

In an embodiment, the kit comprises a third vial comprising a neutralizing solution to adjust pH and/or isotonicity of the radiopharmaceutical solution prior to administration to a patient.

In yet a preferred embodiment, the amount of ²²⁴Ra and ²¹²Pb is in radioactive equilibrium in the first vial.

In yet another preferred embodiment the activity ratio (MBq) between ²¹²Pb to ²²⁴Ra in the first vial is between 0.5 and 2, such as 0.8 - 1.5, or such as 0.8 - 1.3, or such as 0.9 - 1.15.

In yet another embodiment the first vial has a radioactivity in the range 100 kBq to 100 MBq.

In one embodiment of the present invention, the chelator conjugated antibody is added to the ²²⁴Ra (including daughters) solution 30 min to 5 hour before injection, such as 1-3 hours before injection.

In one embodiment of the present invention, the chelator conjugated antibody is added to the ²²⁴Ra (including daughters) solution 1 min to 20 min before injection.

In one embodiment of the present invention, the chelator conjugated antibody is added to the ²²⁴Ra (including daughters) solution 1 min to 10 min before injection.

A kit with a chelate labelled protein or peptide in one vial and a ²²⁴Ra solution in another vial whereby the content of the two are mixed 12 hours to 1 minute before administration also forms part of the invention. In an embodiment, the mixing takes place a few hours (such as 5) to 30 minutes before administration to a patient as to bind ²¹²Pb and or ²¹²Bi to the chelate.
In one embodiment of the present invention, the content of the two are mixed 30 min to 1 hour before injection.

In one embodiment of the present invention, the content of the two are mixed 1 min to 20 min before injection.

In embodiment of the present invention, the content of the two are mixed 1 min to 10 min before injection.

Optionally, a third vial containing a liquid used for dilution and isotonicity adjustment before administration of the radiopharmaceutical solution could be used. This third vial may contain EDTMP, which could chelate ²¹²Bi, if needed.

### Medical uses

The described novel methods and solutions have thereby solved a major problem for the application of ²²⁴Ra in nuclear medicine.

Thus, two types of diseases can be treated with the new formulations:
1. Pure bone related, or sclerotic diseases with radium and daughter nuclide complexed by a phosphonate.
2. Bone-related disease with soft tissue or circulating target cells components by radium and daughter nuclide complexed to chelate-monoclonal antibody conjugate or similar protein or peptide conjugates. A special embodiment of this would be when ²¹²Pb is conjugated to a protein or peptide chelate and the ²¹²Bi is complexed by a bone seeker, *e.g.,* EDTMP.

Proteins or peptides may be used with the current invention including those targeting osteosarcoma, lung cancer, breast cancer, prostate cancer, kidney cancer, thyroid cancer.

Thus, a further aspect of the present invention relates to a radiopharmaceutical solution of the present invention for use as a medicament.

In yet a further aspect of the present invention, the invention relates to a kit according to the present invention, wherein the kit is for use as a medicament.

In yet an embodiment the solution is administered at a dose in the range 50-150 kBq, such as 50-100 kBq per kg of bodyweight.

In yet an embodiment the dosing is the range 8x 10⁹ to 8x 10¹⁰ Ra atoms per kg.

In example 12 (and figure 8) it can be seen that the same effect as for ²²³Ra can be obtained with a much lower dosage (28% lower dosing) of the composition according to the invention in a mice study.

Yet another aspect of the present invention relates to a radiopharmaceutical solution of the present invention for use in treating skeletal disease.

In one embodiment of the present invention, the skeletal disease is selected from the group consisting of skeletal metastases from cancers to the breast, prostate, kidneys, lung, bone, or multiple myeloma, or non-cancerous diseases causing undesired calcification including ankylosing spondylitis.

### Method of production

Herein are described novel methods possible to produce a ²²⁴Ra solution suitable for treating skeletal diseases including primary or metastatic cancer, using centralized production and up to several days of shipment and or storage before administration to patients.

Moreover, such a solution can give higher initial tumor radiation dose compared to a pure ²²⁴Ra solution since the daughter products will give additional dose to tumor when they are made bone-seeking or tumor seeking by the aforementioned chelating complexes. This could make ²²⁴Ra solutions more potent in cancer therapy since the mother nuclide can target the bone disease while the longest-lived daughter nuclide can by an added complex be made to seek out and destroy circulating cancer cells in the blood, alternatively be made a purer bone-seeker by phosphonate complexation.

The novel procedures and methods presented here allows the production and shipment of ²²⁴Ra with longer shelf-life of days or even up to a week or longer since the "problematic" daughter nuclide can be scavenged by tumor-seeking chelates and actually enhance the therapeutic properties of the ²²⁴Ra solutions.

This finding is important since ²²⁴Ra has been considered less useful for *e.g.,* cancer therapy against skeletal metastases because of daughter products with substantial half-lives, in particular ²¹²Pb, which will be present in significant quantities a few hours after the production of a pure ²²⁴Ra solution.

That is made possible by the novel pharmaceutical solutions described here were the ²²⁴Ra targets bone and bone metastases while ²¹²Pb can be made to target circulating tumor cells depending on the chelate-antibody conjugate used. The pharmaceutical solution could be "taylor made" according to the primary tumor from which the skeletal metastases originate. There exist several antibodies with selectivity for different antigens expressed in *e.g.,* prostate-, breast-, lung- , bone-, kidney-, thyroid- and multiple myeloma cancers.

Thus, the invention described herein can be used either as a pure bone-seeker when combined with EDTMP or similar, or if patients have measurable CTC's or is suspected of such, can be used as a combination of treatment of bone metastases and CTC's or soft tissue metastases, thereby adding a new dimension to alpha pharmaceuticals against bone related disease, *i.e.,* an additional prophylactic activity preventing settling of viable CTC's in the skeleton or soft tissues.

Yet another aspect of the invention relates to a method for providing a radiopharmaceutical solution according to the invention, the method comprising:
a) providing a first composition wherein the amount of ²²⁴Ra and ²¹²Pb is in radioactive equilibrium;
b) providing a second composition comprising a complexing agent is selected from the group consisting of acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, and antibody-conjugated-TCMC, wherein the complexing agent is capable of complexing a daughter nuclide of ²²⁴Ra, such as ²¹²Pb, and wherein the complexing agent does not complex ²²⁴Ra; and
c) mixing the first composition and the second composition, thereby providing a pharmaceutical solution according to the invention.

Preferably, the first and second compositions are liquid solutions.

In an embodiment, the activity ratio (Bq) between ²¹²Pb and ²²⁴Ra in the first composition is between 0.5 and 2, such as 0.8 - 1.5, or such as 0.8 - 1.3, or such as 0.9 - 1.15.

In yet an embodiment said complexing agent is selected from the group consisting of EDTMP, antibody-conjugated-DOTA, or antibody-conjugated-TCMC.

In another embodiment said mixing step c) takes place 1 minute to 12 hours before the use as a medicament, such as 30 minutes to 5 hours.

The novel and surprising findings are as follows:
1. Radium solutions can be conditioned with complexing phosphonates or complex conjugated antibodies without causing complexation of radium and causing reduced bone uptake of radium while complexing ²¹²Pb and/or ²¹²Bi *in situ* without a need for purification before use.
2. Daughter nuclides produced during shipment and storage can be complexed effectively *in situ* yielding a radium solution with improved daughter nuclide properties.

The ²²⁴Ra solution can be stored or shipped for several days and still the major fraction of the ²¹²Pb generated can be complexed, *i.e.,* at least 60%, more favorable at least 90%, and even more favorable 95-100%, depending on the amount of complexing agent added.

This is important since it gives a radiopharmaceutical with better overall target to non-target ratios compared to existing radium formulations as shown in following examples and allows the use of ²²⁴Ra solutions that has been shipped and stored for several days. Indeed it teaches away from using freshly prepared ²²⁴Ra solutions with only few hours shelf-life as in the method of Altmann Terapie, since it may be advantageous with the new method that ²¹²Pb has reached equilibrium or close to equilibrium, *i.e.,* at least 1 day or more, to obtain a reproducible and defined ratio between ²²⁴Ra and ²¹²Pb in the administered radiopharmaceutical solution.

We have also confirmed that other complexes than phosphonates can be labeled with daughter nuclide in situ in radium solutions without significantly affecting the radium, e.g., chelate-antibody conjugates were added to radium solution and showed a relevant labeling of daughter nuclide with a significant preservation of the chemical integrity of radium, thus it is possible to have a radium solution whereby one or more of the daughter nuclides is complexed to tumor-seeking molecules yielding a pharmaceutical solution with bi-specific targeting properties, *e.g.,* radium targeting the skeletal disease and a chelate complex with daughter nuclides targeting a tumor cell antigen on circulating tumor cells etc. As a special embodiment one daughter nuclide can be complexed to a monoclonal antibody and the other to a phosphonate.

In a special embodiment of the current invention is a solution with radium containing TCMC conjugated to a protein or a peptide, preferentially a monoclonal antibody, whereby the antibody is targeting an antigen on breast-, prostate-, lung-, kidney-, bone- or multiple myeloma cancer cells. If left for a few minutes to a few days, the ²¹²Pb generated will mainly bind to the TCMC-antibody conjugate. When such a solution is injected into a cancer patient, ²²⁴Ra will protect the bone from destruction by killing tumor cells at the bone surface and in the skeleton and the ²¹²Pb labeled antibody will kill circulating cells out of range from the radium radiation. Thus, what was before a problem of free daughter nuclides with undesired biodistribution is made into an advantage for the ²²⁴Ra series by the use of the current invention. It is particularly attractive to use an internalizing antigen as target since ²¹²Pb-conjugates are especially effective as *in vivo* generator for alpha-emitters under such conditions whereby the alpha emitting ²¹²Bi daughter is enclosed in the target cells (Boudousq *et al.,* 2013).

In one embodiment the radiopharmaceutical product is shipped ready to use, *e.g.,* in a vial with a septum for withdrawal of the solution with a syringe or even as a prefilled syringe ready to use. In a second embodiment, the product may be shipped in a kit format consisting of a vial with ²²⁴Ra solution, a second vial with a solution of a chelator and optionally a third solution with a formulation buffer for the adjustment of concentration and/or pH etc. The chelator, whereby it is a phosphonate, chelator-antibody conjugate or similar, is added to the Ra solution and mixed for a few minutes to a few hours most preferable from 5 -60 minutes, before the product is, if needed added a formulation buffer, and administered to the patient.

### Examples

### Example 1. Calculation of the ²¹²Pb daughter nuclide level from ²²⁴Ra decay at various time points.

### Background

The ²¹²Pb produced after preparation of a pure ²²⁴Ra radiopharmaceutical may be a problem since it has different and undesirable properties compared with the mother nuclide. *E.g.,* it is known that radium can target bone and bone metastases, but the lead progeny has undesired accumulation in hematopoietic cells and tissues and in the kidneys.

### Method

The ingrowth of ²¹²Pb from a pure ²²⁴Ra source was calculated using a universal activity calculator.

### Results

Figure 5 shows the amount of ²¹²Pb at various time points after the production of a pure ²²⁴Ra pharmaceutical solution and storage in a gas tight container.

### Conclusion

The data shows that significant amount of daughter nuclide is present within a relatively short time frame, complicating a potential centralized production and supply of ²²⁴Ra based radiopharmaceuticals. It is noteworthy though that the ratio of ²¹²Pb to ²²⁴Ra in the solution reaches 1 after 36 hours and thereafter gradually increases to about 1.1 of which it stay for the rest of the time until complete decay.

### Example 2: Preparation of radionuclides and counting of radioactive samples.

In the following, all work with the concentrated radioactive preparations including evaporation of solvent etc was performed in a glove-box. A source of ²²⁸Th in 1 M HNO₃ was acquired from a commercial supplier. Ac-resin was obtained from Eichrom Technologies LLC (Lisle, IL, USA) in the form of a pre-packed cartridge. To use smaller volume of solvent, about thirty percent of the materials in a cartridge (Cartridge 1) was extracted and repacked in a smaller column (Cartridge 2) made by a 1 ml filtration column (Isolute SPE, Biotage AB, Uppsala, Sweden). A slurry representing 20% of the original cartridge content was used for immobilizing of ²²⁸Th in 500 mikroliter 1 M HNO3 which was added 500 microliter of 1 M HCl and incubated by shaking the vial (4 ml vial, E-C sample, Wheaton, Millville, NJ, USA) for at least 4 hours. Cartridge 2 was added a small amount (about 0.1 ml) of the Ac-resin. Thereafter, the slurry was added to cartridge 2 using the prefilled material as a catcher layer. Radium could be eluted from the Cartridge 2 in 2 ml of 1 M HCl. The 2 ml radium solution was evaporated to dryness, using a heater block and flushing the vial with N2 gas through a Teflon tube inlet and outlet in the rubber/Teflon septum on the vial and by leading the acid vapor into a beaker of saturated NaOH by a stream of N₂-gas.

The residue was resolved in 0.5 ml 1 M HNO₃ and loaded to a cartridge 3 consisting of a 1 ml Isolute column packed with about 250 mg Dowex cation exchanger. Cartridge 3 was washed with 7 ml 1 M HNO₃, which removed ²¹²Pb, and finally with 3-4 ml 8 M HNO₃ to elute ²²⁴Ra. The ²²⁴Ra eluate was evaporated to dryness, using the heater block and a flow of N₂-gas, and the residue could be dissolved in 0.1 M HCl. Typically, more than 70% of the ²²⁴Ra present in the ²²⁸Th source could be extracted and purified using the described methods.

Radioactive samples were counted on a Cobra II Autogamma counter (Packard Instruments, Downer Grove, IL, USA). During extraction of ²²⁴Ra from the ²²⁸Th source, a CRC-25R dose calibrator (Capintec Inc., Ramsey, NJ, USA) was used.

To determine distribution of ²²⁴Ra, ²¹²Pb and ²¹²Bi in real time in samples, a liquid nitrogen cooled HPGe detector (GWC6021, Canberra Industries, Meriden CT, USA) was used. This was combined with a DSA 1000 digital signal analyzer and the Genie 2000 software (Canberra).

### Example 3: Determining net count rate for ²¹²Pb in a ²¹²Pb/²²⁴Ra mixture before radioactive equilibrium has been reached.

After more than 3 days, *i.e.,* "equilibrium" a sample will for practical purposes have 1.1 times ²¹²Pb vs ²²⁴Ra.

Regardless of whether ²¹²Pb is higher or lower than equilibrium it can be assumed that this is reached after 3 days since surplus ²¹²Pb is reduced by 99% and the ingrowth of ²¹²Pb from ²²⁴Ra is practically complete vs. "equilibrium".

Using the Cobra II Autogamma counter with a counting window setting from 70-80 KeV gives mainly the ²¹²Pb with very little contribution from other radionuclides in the ²²⁴Ra series. Radium-224 must be indirectly counted when the initial ²¹²Pb has vanished and equilibrium between ²²⁴Ra and ²¹²Pb has been reached (after approximately 3 days). This indirect counting requires the sample to be stored in a relatively gas tight containers as otherwise the ²²⁰Rn may escape preventing the radionuclide equilibrium of 1.1 between ²¹²Pb and ²²⁴Ra to be reached.

Since sampling and counting may be separated by some time, the net count rate for ²¹²Pb can be adjusted for decay to determine the net ²¹²Pb count rate at the time of sampling.

### Example 4: Thin layer chromatography analyses

Thin layer chromatography (TLC) was performed using chromatography strips (model # 150-772, Biodex Medical Systems Inc, Shirley, NY, USA). A small beaker with about 0.5 ml of 0.9% NaCl was used to place strips with a sample spot in. To the strip was typically added 1-4 µl of sample at approximately 10% above the bottom of the strip. After the solvent front had moved to about 20% from the top of the strip, the strip was cut in half and each half was placed in a 5 ml test tube for counting. In this system radiolabeled antibody and free radionuclide does not migrate from the bottom half while radionuclide complexed with EDTA migrates to the upper half. A formulation buffer (FB) consisting of 7.5% human serum albumin and 5 mM EDTA in DPBS and adjusted to approximately pH 7 with NaOH was mixed with the antibody conjugates in ratio 2:1 for at least 5 minutes before application to the strips to determine free radionuclide.

The analysis of the EDTMP was done without the formulation buffer (FB) (Figure 6A,). The labelling of radionuclide with EDTMP was measured by the amount of migration to the upper half of strips. The DOTMP migrated poorly in this system and therefore these solutions had to be treated with FB to measure free radionuclide at the upper half of the strip (Figure 6A).

### Conclusion

It is shown from the thin layer analysis that modest amounts of EDTMP and DOTMP of approximately 0.011 - 0.012 mM can complex ²¹²Pb in a similar manner as high amounts of EDTA of approximately 3.3 mM in a ²²⁴Ra solution.

### Example 5: In situ chelation of ²¹²Pb in ²²⁴Ra solutions.

Initially ²²⁴Ra solutions in 0.1 M HCl was neutralized with 1 M NaOH and the EDTMP was added to the solution to obtain a pH slightly below neutral. In later experiments, 10:1 ratio of ²²⁴Ra in 0.1 M HCl and 5 M ammonium acetate was used before addition of the chelators, resulting in a pH range of 5.5 - 7 for the reactions. Reaction times of 30 minutes to several days, at room temperature, were tested for EDTMP with good labelling yield (typically above 90% according to TLC) when concentrations of about 4-8 mg/ml EDTMP in reaction solutions were used. Thus, EDTMP seems to be a good scavenger for ²¹²Pb *in situ* in ²²⁴Ra solutions. As for DOTMP the labelling was less efficient with about 70% labelling for 7 mg/ml at room temperature and about 1 h of reaction time. The labelling yield of DOTMP may be improved by adjusting chelator concentration or reaction time etc. It should be noted that subsequent experiments using EDTMP and ammonium acetate buffers showed a good labelling with ²¹²Pb also in radium solutions of pH 5.5 to 7 as determined by thin layer chromatography (Figure 6A).

Long-term scavenging was tested by leaving a ²²⁴Ra solution with EDTMP (approximately 6 mg/ml) buffered to about pH 6 with ammonium acetate, for 7 days at room temperature. Analysis of the ²¹²Pb distribution profile was performed by use of TLC as described.

### Results

At least 93% of the activity was found in the upper half of the TLC-strip corresponding to EDTMP associated activity after 7 days.

### Conclusion

It is possible to effectively chelate ²¹²Pb generated *in situ* in ²²⁴Ra solution by EDTMP. Thus, it is possible to prepare ready to use ²²⁴Ra solutions with a bone-seeking chelator that scavenge ²¹²Pb *in situ* thereby obtaining ²²⁴Ra solution with improved shelf life for use as a bone targeting radiopharmaceutical.

As an alternative, a labelling kit may be used whereby EDTMP is added to a several days old ²²⁴Ra solutions a few minutes to a few hours before administration. Such a labelling kit will also allow centralized production of ²²⁴Ra as the kit can be very simple to use for ²²⁴Ra solutions several days to more than a week after the production date for ²²⁴Ra.

In an experiment with an 8 day old ²²⁴Ra solution in 0.1 M HCl and 0.5 M ammonium acetate, EDTMP to a concentration of about 7 mg/ml was added. After 10 minutes and 1 hours standing at room temperature, TLC analyses showed 91% and 93%, respectively, of the ²¹²Pb was associated with the EDTMP.

EDTMP solutions up to 4 months old were tested and found to be functional, thus EDTMP seems well suited to be used in a kit format.

### Example 6: Biodistribution in mice of ²²⁴Ra with significant amounts of ²¹²Pb with and without EDTMP.

### Background

The main objective was to study the biodistribution of the injected radionuclides with or without EDTMP. An EDTMP containing- and a saline control solution, respectively, of ²²⁴Ra in equilibrium with daughter radionuclides were used. Materials and methods: Animal experiments were performed according to European regulations for animals used for scientific purposes. Nude mice were fully grown and were at an age of more than 6 months. A 3 day old 0.1 M HCl solution containing ²²⁴Ra was split in two. One fraction was added EDMP and 1 M NaOH to adjust the pH to approximately 8, to a final concentration of 5 mg EDTMP per ml (solution A). The other fraction was adjusted by 1 M NaOH to approximately pH 7 (solution B). Each solution was sterile filtered through a 13 mm 0.2 µm Acrodisc syringe filter (Pall Life Science, Port Washington, NY, USA) with Supor membrane. Thereafter 100 µl with approximately 20 kBq of ²²⁴Ra were administered by tail vein injection into each mouse.

### Results

As shown in Figures 3A and 3B there was a significant improvement in ²¹²Pb distribution when EDTMP was added. The soft tissue and blood uptake was significantly reduced while the bone targeting was similar as free ²¹²Pb. Thus, the bone to soft tissue ratios were greatly improved for ²¹²Pb by adding EDTMP to the ²²⁴Ra solution. There is no significant change in the ²²⁴Ra distribution when adding EDTMP as shown in Figure 3A and 3B.

### Conclusion

Addition of EDTMP to ²²⁴Ra solutions improves ²¹²Pb biodistribution with no significant changes to the ²²⁴Ra biodistribution.

### Example 7: Labelling of chelator-conjugated antibody with ²¹²Pb in situ in ²²⁴Ra solution.

### Background

It is advantageous from a logistic perspective that radiopharmaceutical can be produced in a centralized production unit and shipped to the end user. The ²¹²Pb generated should be scavenged by the chelator to minimize injection of free ²¹²Pb when ²²⁴Ra is used.

### Methods

²²⁴Ra was produced and purified as described in example 2. TCMC- and DOTA-labeled monoclonal antibodies were prepared by using antibodies purified with centrifuge concentrator (Vivaspin 4 or 20, 50 000 MWCO, Sartorius Stedim, Goettingen, Germany) and added 150 mM carbonate buffer, pH 8.5-9. The antibody had a concentration of typically 20-30 mg/ml and was added p-SCN-Bn-TCMC or p-SCN-Bn-DOTA (Macrocyclics Inc, Dallas, Tx, USA) using antibody to chelator ratios of 1:9 or 1:5, respectively. After at least two hours incubation at room temperature the reaction was terminated by adding 0.1 M glycine in carbonate buffer (pH approximately 8.5) and further incubation for 10 minutes before purification and buffer exchange into 0.9% NaCl using centrifuge concentrator (Vivaspin). Chelator-antibody concentrations of 15-35 mg/ml in 0.9% NaCl were used as stock solutions.

To a 2 ml Eppendorf tube was added typically 40 µl ²²⁴Ra in 0.1 M HCl, 5 µl of 5 M ammonium acetate, 5-10 µl, (15-30 mg/ml) TCMC- or DOTA-labeled antibody in 0.9 % sodium chloride. This method was tested for 4 different antibody conjugates including those of trastuzumab (Herceptin), rituximab, cetuximab, and OI-3 murine monoclonal antibody. The pH was determined to be in the range of 5.4-6.0 by applying 1 µl on a pH paper (No 1.09564.0003 and 1.09556.003 from Merck KGaA, Darmstadt, Germany) and reading the colour. The reaction was performed at room temperature.

In some of the experiments a control solution with the same ingredients except for that the antibody did not contain TCMC or DOTA was reacted in parallel using the same conditions. After 30 and 100 minutes 5 µl was withdrawn and mixed with 10 µl of a formulation buffer (FB) consisting of 7.5 % human serum albumin and 5 mM EDTA in DPBS. After at least 10 minutes, 1-4 µl of the product/FB mixture was withdrawn and placed on a thin layer chromatography strip (Biodex). The same procedure was performed with the control solution. The strips were eluted in 0.9% NaCl solution and when the solvent front reached almost to the top, the strip was removed, cut in half and the bottom and top part was counted separately on a Cobra II gamma counter (as previously described).

### Results

The thin layer profiles are presented in Figure 6B. Typically more than 90% of the activity was found at the bottom half of the thin layer strip for the TCMC- and DOTA-antibody conjugate. In the control which included formulation buffer (FB) with EDTA (Figure 6A,), typically more than 97% of the activity was found in the top half of the strip signifying that the ²¹²Pb was free to be complexed by EDTA. This shows that both TCMC- and DOTA-conjugated antibodies can be efficient scavengers for ²¹²Pb in ²²⁴Ra solution. In conclusion, it is possible to use TCMC or DOTA-antibody conjugate to scavenge ²¹²Pb in ²²⁴Ra solution thus, enabling the production of a pharmaceutical solution with dual targeting properties, *i.e.,* a bone seeking ²²⁴Ra and an antigen seeking ²¹²Pb conjugate.

In a follow-up experiment a ²²⁴Ra solution, added TCMC-labeled chOI-3 (chimeric OI-3) monoclonal antibody conjugate (to about 1.5 mg/ml), buffered to about pH 5.5 with ammonium acetate and kept for 7 days at room temperature. Thereafter samples were withdrawn and mixed 1:2 with formulation buffer (as described) and after 5 minutes or more applied on TLC strips as described. It was found that on average 95.6% was retained with the protein (lower half of the strip).

### Conclusions

²¹²Pb is scavenged/complexed effectively *in situ* in ²²⁴Ra solution over several days by TCMC-labeled antibody. Thus, it shows that centralized production requiring storage and shipment for several days is possible for ²²⁴Ra solutions with chelate-antibody conjugate.

### Example 8: Cell binding experiment with radiolabeled monoclonal antibody in mixture with ²²⁴Ra

### Background

The human osteosarcoma cell line OHS expresses Her-2 (relatively weak) and MUC-18 (moderate). They were therefore used for evaluating cell-binding fraction of chelator conjugated trastuzumab and chOI-3 antibodies against Her-2 and MUC-18, respectively. Radium-224 was dissolved in 0.1 M HCl and left for two days to reach equilibrium with ²¹²Pb and ²¹²Bi. To adjust pH, 12 µl of 5 M ammonium acetat in metal free water was added to 100 µl of ²²⁴Ra in 0.1 M HCl and thereafter added 200 µg of TCMC-labeled trastuzumab. After 30 minutes thin layer chromatography confirmed that more than 90% of the ²¹²Pb was scavenged by the chelator. The reaction mixture was sterile filtered using a 13 mm syringe filter and the product tested for cell binding using approximately 10 million cells in 0.2 ml of DPBS with 0.5% BSA. Cells were either blocked by incubation with 20 µg of the same antibody for 15 minutes (to measure non-specific binding) or left unblocked before adding reaction solution with about 10 ng of chelate-antibody conjugate mixed with radionuclide to each tube. After 1 hour of incubation, tubes were counted on the Cobra II gamma counter to determine added activity. Thereafter the cells were washed three times with 0.5 ml DPBS/0.5% BSA by whirlmixing, centrifugation and removal of supernatant and then the cell bound activity in the tubes were measured. The percent of cell bound antibody conjugate was determined as bound after wash divided by the added activity times 100.

### Results

When corrected for decay and radiochemical purity and the non-specific binding was subtracted, it was found that 64.3 -72.2% of the ²¹²Pb-labeled antibodies were bound specifically to the cells. In this one point assay, this indicates relevant targeting properties of the ²¹²Pb-antibody conjugate produced *in situ* in ²²⁴Ra solutions.

### Conclusion

It is shown that ²¹²Pb-labeled conjugates with relevant tumor targeting properties can be produced *in situ* in ²²⁴Ra solutions.

### Example 9: Biodistribution in mice of ²²⁴Ra/ ²¹²Pb with TCMC-labeled monoclonal antibody.

### Background

To study whether ²²⁴Ra/²¹²Pb solutions could be used for preparing skeletal targeted and tumor cell targeted co-therapeutics.

### Materials and Methods:

A one day old ²²⁴Ra solution as described in example 6 was added NaOH, 5 M ammonium acetate and TCMC-labeled Trastuzumab in the same way as in example 7 and stored over night. The solution was added metal free water in a 1:1 ratio, sterile filtered using a 13 mm 0.2 µm Acrodisc syringe filter (Pall Life Science, Port Washington, NY, USA) with Supor membrane. The cell binding ability of the ²¹²Pb-labeled antibody component in the ²²⁴Ra solution was verified as in example 8. Animal experiments were performed according to European regulations for animals used for scientific purposes. Animals were euthanized and blood drawn from the heart before they were dissected. Urine, blood and tissue samples were placed in 5 ml tubes. The weight of the tubes were measured before and after addition of samples to determine exact sample weight. The radioactivity content were measured in the Cobra gamma counter. Samples were counted shortly after the dissection and again after 3-4 days when radioactive equilibrium had been reached to determine ²¹²Pb and ²²⁴Ra content, respectively.

### Results

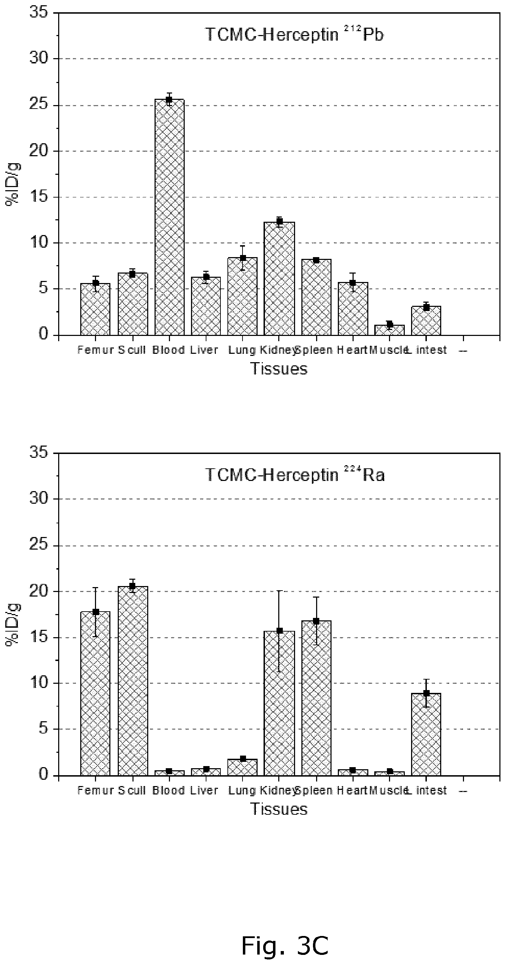

The bio-distribution profiles are shown in Figure 3C. The ²¹²Pb showed a distribution profile as expected for a radiolabeled antibody, *i.e.,* high activity in blood and blood rich tissues and low activity in femur and scull. Compared with free ²¹²Pb (Figure 3 A), the TCMC- trastuzumab (Herceptin) conjugated ²¹²Pb had significantly less uptake in femur and scull while the activity level in blood and blood rich organs were higher. It should be noted that the quality of the distribution is different in blood for free ²¹²Pb and ²¹²Pb-TCMC-herceptin as the latter is circulating with a slow blood clearance but not taken up in the blood cells as with the free ²¹²Pb. The ²²⁴Ra showed high uptake in femur and scull and low uptake in blood and was very similar to that found in the biodistribution of chelator free radium solution (Figure 3A).

In Figure 7 the uptake ratios for bone vs. blood and bone vs. kidney for ²¹²Pb, ²¹²PB-EDTMP and ²¹²Pb-TCMC-trastuzumab in ²²⁴Ra solutions are presented. The ratios show improved bone to blood and bone to kidney ratios for ²¹²Pb-EDTMP compared with non-complexed ²¹²Pb. For ²¹²Pb-TCMC-herceptin, however, the bone to blood ratios were lower than for free ²¹²Pb. This is expected since macromolecular sized monoclonal antibodies clear slowly from the blood compared with most small molecular weight compounds. This may be an advantage when targeting circulating tumor cells as the increased residence time in blood enhances the probability of binding to target cells in circulation. It should also be noted that for short radiation range alpha particles the effect of cellular bound radionuclide may be much stronger than that of circulating radionuclide because of the short proximity to the DNA for cell associated- vs. the freely circulating radionuclide.

### Conclusion

Chelator labelled monoclonal antibody can effectively scavenge ²¹²Pb without reducing the bone seeking properties of ²²⁴Ra in radiopharmaceutical solutions containing the two radionuclides. Thus, it shows the possibility of obtaining dual targeting properties of ²²⁴Ra/²¹²Pb by adding complexing agents to the solutions, thereby strengthening the therapeutic potential of the radiopharmaceutical solution and reducing possible undesired uptake of ²¹²Pb in hematopoietic cells and tissues while upholding the bone seeking properties of ²²⁴Ra.

### Example 10: A kit system for avoiding radiolysis

Because of the highly localized nature of alpha-irradiation radiolysis must be considered as a potential problem and the radiopharmaceutical must be designed to minimize this. According to the knowledge in the field radiolabeled antibodies are sensitive to radiolysis and therefore a kit system may be advantageous for ²²⁴Ra solutions which are to be combined with chelator conjugated antibodies for scavenging ²¹²Pb. When ²²⁴Ra is in equilibrium with progenies it produces approximately 28 MeV per decay of the complete series. Thus, a solution of 1 MBq/ml contains N = A/λ = 10⁶ s-1 /(0.693/[3.64 × 24 × 3600 s]) = 4.53 × 10¹¹ atoms of ²²⁴Ra where N is the number of atoms and A is activity in Bq and λ is the decays constant which is equal to In2/t_{1/2} and t_{1/2} is the half-life for ²²⁴Ra.

The radiation dose, D, is defined as energy per mass, *i.e.,* J/kg in SI-units.
For the complete decay of 1 MBq of ²²⁴Ra in 1 ml of aqueous liquid it would amount to D = (4.53 × 10¹¹ × 28 MeV × 1.6 × 10⁻¹³ J/MeV)/10⁻³ kg = 2029 Gy that is, in one half-life of 3.64 days a solution of 1 MBq/ml of ²²⁴Ra in equilibrium with daughters will be exposes to about 1 kGy of self-irradiation. For a monoclonal antibody it is usually advisable be keep the self dose of the radiopharmaceutical solution below 0.5 kGy to avoid reduced binding properties due to radiolysis.

Thus, a kit system whereby chelator conjugated antibody is added, e.g., by a syringe to a vial containing the ²²⁴Ra (including daughters) solution a few hours to a few minutes before the product is administered to a patient is advised for concentrated solutions of ²²⁴Ra with progenies suitable for long distance shipment.

Example of scavenging ²¹²Pb with TCMC-monoclonal antibody conjugate in a 7 days old ²²⁴Ra solution. A ²²⁴Ra solution produced one week earlier was added 10% ammonium acetate and TCMC-rituximab (to a final concentration of about 5 mg/ml) to a final volume and pH of about 0.1 ml and 5.5, respectively, and kept at room temperature overnight. After 18 hours a sample was withdrawn and mixed with formulation buffer as described. TLC analyses showed that 91% of the ²¹²Pb activity was protein bound, *i.e.,* in the lower half of the TLC strip as determined by gamma counting. This demonstrates that a kit with a vial containing a ²²⁴Ra solution and a separate vial with a chelator-labeled protein or similar can be combined and used to scavenge ²¹²Pb in the ²²⁴Ra several days after ²²⁴Ra production date, thereby preparing a ²²⁴Ra bone-seeker with a ²¹²Pb tumor- seeking complex using short reaction times to avoid radiolytic degradation of the product.

It was verified that the reagents, other than ²²⁴Ra, could be stored several weeks or months without losing their function, thus, they are well suited for use in a kit format.

### Conclusion

A simple kit format would make it possible to use ²²⁴Ra solutions in equilibrium with ²¹²Pb to locally prepare dual targeting radiopharmaceutical with bone seeking radium and tumor cell seeking ²¹²Pb-antibody conjugate based on centralized produced ²²⁴Ra allowing for long-distance shipment take up to several days while avoiding radiolysis of radioimmunoconjugate by adding antibody conjugate before administration to the patient.

### Example 11: Circulating tumor cells

Circulating tumor cells could give rise to new tumor lesions in bone or soft tissues and may be addressed by the novel radiopharmaceutical solution described herein.

One may argue that the amount of ²¹²Pb in therapeutic ²²⁴Ra solution may be moderate to modest (*i.e.,* at equilibrium about 1.1 times that of ²²⁴Ra). If one assume similar dosing of ²²⁴Ra as is done with ²²³Ra in patients but correct for the half-life difference, roughly 150 kBq per kg of bodyweight would be the administered dose. This is just an example and a dosing may differ significantly according to disease and what level of side effects that is acceptable.

At equilibrium 150 kBq per kg of body weight would translate into a ²¹²Pb-antibody conjugate dosage of about 11.5 MBq in 5 litre of blood in a 70 kg patient. The number of circulating tumor cells is typically less than 10 cells per ml thus in 5 l blood there are less than 50 000 tumor cells in total. If only 1 in 100 000 of the injected ²¹²Pb-antibody conjugate binds to the tumors cells this would mean 0.0023 Bq per cell, equivalent to 127 ²¹²Pb atoms bound per cell, which would be highly destructive as it has been reported that a mean of 25 cell bound ²¹²Pb per cell would kill 90% of a cell population.

The number of atoms bound to a cell will depend on the specific activity of the ²¹²Pb-conjugated antibody and the number of antigens available at the target cells. Lead-212 labeled TCMC-trastuzumab with a specific activity of about 37 MBq/mg (1 mCi/mg) was recently evaluated in a clinical study.

A ²¹²Pb-labeled monoclonal antibody with a specific activity of 37 MBq per mg has a ²¹²Pb atom to antibody molecule radio of 1:1973, that is, very few of the antibody molecules are actually radiolabeled. To reach a 90% cell kill level of ²¹²Pb of 25 atoms per cell, one would need to bind 49325 antibody molecules per cell. This is obtainable as several tumor associated antigens are expressed at levels exceeding this. Also from a chemical stand point this is plausible as it is possible to conjugate typically 1 to 5 chelator units per antibody molecule without losing the antigen binding properties, so a very small fraction of the chelator-groups are actually occupied by ²¹²Pb during and after the radiolabeling.

*Conclusion:* The ²¹²Pb-labeled antibody in a dual targeting ²²⁴Ra radiopharmaceutical can be made with a strong therapeutic potential against circulating tumor cells at ²²⁴Ra levels suitable for treating bone tumors.

### Example 12 - Mice in vivo studies

### Background

To underline the superiority of the composition comprising un-complexed ²²⁴Ra and bone-seeking EDTMP-complexed ²¹²Pb, a study was conducted showing the effect of bone-seeking ²¹²Pb-EDTMP + ²²⁴Ra mixture (Example 12A), and compared it to the effect of the FDA approved drug Xofigo (²²³Ra) (Example 12B). A comparison of the data from Examples A and B are shown in Figure 8.

### Example 12A

### Background and methods:

The antitumor activity of bone-seeking agents can be tested in animal models of skeletal metastases. EDTMP (control solution) and ²²⁴Ra + ²¹²Pb-EDTMP (test solution) were produced and stored to achieve equilibrium between ²²⁴Ra and ²¹²Pb in the latter before administration. Test and control solutions were shipped to Pharmatest Services Ltd, Finland, a contract research organization (CRO), and tested at their animal facility. The breast cancer model MDA-MB-231 (SA) in nude mice was used. This model produces bone lesion and osteolysis due to skeletal metastases, and later on soft tissue metastases as well. Four to five weeks old female nude mice were inoculated with 10⁵ cells in 0.1 ml PBS at day 0. On day 2, either 0.9% NaCl or 25 µg/kg of bodyweight EDTMP was administered to control groups, and 45, 91 and 179 kBq/kg of ²²⁴Ra equilibrated with ²¹²Pb EDTMP-complexed was administered to three treatment groups. Each group had 12 animals. Animals were sacrificed when symptom of tumors (*e.g.,* paraplegia, cachexia, body weight loss of 20% or more or breathing difficulty) were observed. Analgesic treatment was used individually during the last study days when needed.

### Results

Animals in the control groups showed median survival of 22 and 23 days respectively. There was no significant difference between the two control groups. The treatment groups had median survival of 25, 28 and 31 days for 45, 91 and 179 kBq/kg of ²²⁴Ra/²¹²Pb-EDTMP, respectively. All three treatment groups had statistically significant life prolongation as compared to the control groups. The tumor burdens at bone sites at the time of sacrificing the mice were significantly reduced in ²²⁴Ra/²¹²Pb-EDTMP treated mice as compared to control mice. Osteolytic area at the time of sacrifice was significantly reduced for the 91 and 179 kBq/kg ²²⁴Ra/²¹²Pb-EDTMP treatment groups as compared to EDTMP control group.

### Example 12B

Pharmatest Services Ltd, Finland, the same CRO, had previously used the same breast cancer model MDA-MB-231 (SA) in nude mice to study the effect of ²²³Ra (Xofigo), a FDA approved compound. See, Survival benefit with radium-223 dichloride in a mouse model of breast cancer bone metastasis. Suominen et al., J Natl Cancer Inst, 2013 Jun 19, 105(12):908-16, doi: 10.1093/jnci/djt116, Epub 2013 May 16.

It should be noted in the following: Because of the difference in the half-lives of ²²⁴Ra (3.6 days) and ²²³Ra (11.4 days), 1 Bq of ²²³Ra represents about 3.2 times the number of radium atoms compared with 1 Bq of ²²⁴Ra.

When comparing the results from Examples A and B (Figure 8), it is evident that despite the dosing with ²²⁴Ra atoms (8.6 x 10¹⁰) being only 24% of the dosing with ²²³Ra atoms (3.6 x 10¹¹), the survival advantage conferred by ²²⁴Ra + ²¹²Pb-EDTMP (41%) was similar to the survival advantage conferred by ²²³Ra (43%). In addition, it is noted that the similar survival advantage was achieved with a 28% lower dosing in terms of kBq/kg of ²²⁴Ra/²¹²Pb-EDTMP (179 kBq/kg) as compared to ²²³Ra (250 kBq/kg).

Furthermore, it is noted that both mother nuclides ²²³Ra and ²²⁴Ra (including their daughter nuclides) produce 28-29 MeV of radiation during complete decay and produce four alpha-particles each. Despite of these similarities in decay properties, the number of Ra atoms (atomic dosing) and the activity dosing required for producing similar survival benefit for ²²⁴Ra/²¹²Pb-EDTMP is only 24% and 72%, respectively of that required for ²²³Ra (when both mother nuclides are in equilibrium with their daughter nuclides). Thus, the ²²⁴Ra + ²¹²Pb-EDTMP solution showed an unexpectedly high antitumor activity at a much lower dosing both in terms of Ra atoms and radioactivity per body weight as compared with ²²³Ra solution used by Suominen *et al.*

### Conclusion

The results in example 12 demonstrate a therapeutically promising and unexpected high effect from low doses of of ²²⁴Ra + ²¹²Pb-EDTMP compared to the present FDA-approved gold standard in the field (Xofigo), by showing similar survival benefit effects with only 24% of the radioactive atoms and only 72% of the radioactivity dosing as compared to ²²³Ra. In addition to the therapeutic benefits, this unexpected effect has several other important features including, for example, in relation to side effects, risk during handling, etc. Furthermore, by using ²²⁴Ra instead of ²²³Ra, a patient would be exposed to radioactivity for a shorter period following treatment because the half-life of ²²⁴Ra is less than one-third that of ²²³Ra. Thus, the composition of the present invention presents unexpected results over the current benchmark of treatment.

### Example 13: Measurement of complex properties using EDTMP in a solution of ²²⁴Ra in equilibrium with ²¹²Pb.

### Background

A several days old ²²⁴Ra solution was added EDTMP to 5 mg/ml and the binding of the radionuclides on a cation exchanger was evaluated.

### Method

A 1 ml Isolute column was packed with about 250 mg Dowex cation exchanger (50 W X 8, hydrogen form, Sigma-Aldrich). The ion exchange column was washed with 1 M NaOH and later 0.9% NaCl until eluate had a pH of about 7. This would make it possible to keep the eluate approximately to neutral pH. Thereafter, the radium solution was added to the cation exchanger and eluted with 4 ml 0.9% NaCl solution. The eluate was collected in 4 tubes with 1 ml in each. Thereafter the tubes and the cation exchanger columns was counted immediately on the gamma counter for ²¹²Pb determination and recounted later for ²²⁴Ra determination as described in example 3. For the ²¹²Pb more than 95% eluted with the saline solution indicating complexation with EDTMP. The ²²⁴Ra was quantitatively retained on the cation exchanger, *i.e.,* with less than 1 % eluted with the saline solution. When a control solution without EDTMP was eluted though the cation exchanger the major fraction of the ²¹²Pb was retained on the cation exchanger indicating that the strong elution of ²¹²Pb with EDTMP was due to complexation.

### Conclusion

Radium-224 was strongly retained on a cation exchanger while ²¹²Pb was readily eluted when a solution of EDTMP mixed with radionuclide was loaded onto the cation exchanger and eluted with isotonic saline. This shows that ²²⁴Ra retain its free cationic properties in an EDTMP solution capable of complexing daughter nuclide.

### Example 14. Complexation properties of TCMC- or DOTA-labeled monoclonal antibody in mixture with ²²⁴Ra and ²¹²Pb assessed by size selective centrifuge microconcentration cartridge.

Using a centrifuge filtering unit with a cut-off of 30 kDa (Vivaspin 4 or 20, Sartorius Stedim, Goettingen, Germany) it was shown that ²²⁴Ra in a solution could be separated effectively from the TCMC- or DOTA conjugates by washing with 0.9% NaCl solution. By starting with 2 ml and concentrate to approximately 0.25 ml > 85% of the radium was removed from the ²¹²Pb-antibody conjugate in the concentrate with a high retention > 80% ²¹²Pb in the concentrate, thus it shows that TCMC- and DOTA-antibody conjugates can scavenge/complex ²¹²Pb without significantly complexing ²²⁴Ra.

### Conclusion

Assessment using microconcentration units which concentrate high molecular weight compounds including radiolabeled monoclonal antibody, indicated that ²¹²Pb was complexed to chelator-monoclonal antibody while ²²⁴Ra was not retained by the complexing agent.

### References

Jaggi JS, Kappel BJ, McDevitt MR, Sgouros G, Flombaum CD, Cabassa C, Scheinberg DA. Efforts to control the errant products of a targeted in vivo generator. Cancer Res. 2005 Jun 1;65(11):4888-95.

Jones SB, Tiffany LJ, Garmestani K, Gansow OA, Kozak RW. Evaluation of dithiol chelating agents as potential adjuvants for anti-IL-2 receptor lead or bismuth alpha radioimmunotherapy. Nucl Med Biol. 1996 Feb;23(2):105-13.

Nilsson S, Larsen RH, Fossa SD, Balteskard L, Borch KW, Westlin JE, Salberg G, Bruland OS. First clinical experience with alpha-emitting radium-223 in the treatment of skeletal metastases. Clin Cancer Res. 2005 Jun 15;11(12):4451-9.

Piotrowska A. et al."Functionalized NaA nanozeolites labeled with 224,225Ra for targeted alpha therapy",JOURNAL OF NANOPARTICLE RESEARCH, vol. 15, no.11,31 October 2013.

## Claims

1. A radiopharmaceutical solution comprising uncomplexed ²²⁴Ra and complexes between a complexing agent and ²¹²Pb;
wherein said complexing agent is selected from the group consisting acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, TCMC, DOTA, p-SCN-Bn-DOTA, p-SCN-Bn-TCMC, antibody-conjugated-DOTA, and antibody-conjugated-TCMC.

2. A radiopharmaceutical solution comprising ²²⁴Ra, ²¹²Pb and a complexing agent selected from the group consisting of EDTMP, antibody-conjugated-DOTA, or antibody-conjugated-TCMC.

3. The radiopharmaceutical solution according to claim 1 or 2, wherein the complexing agent is capable of complexing at least ²¹²Pb.

4. The radiopharmaceutical solution according to any of the preceding claims, wherein the complexing agent is capable of complexing a daughter nuclide of ²²⁴Ra, such as ²¹²Pb, in the pharmaceutical solution.

5. The radiopharmaceutical solution according to any of the preceding claims, wherein the complexing agent does not complex ²²⁴Ra in the pharmaceutical solution.

6. The radiopharmaceutical solution according to any of the preceding claims, wherein the complexing agent is conjugated to a compound selected from the group consisting of a monoclonal antibody, a vitamin, a polyclonal antibody, an antibody fragment, a synthetic protein, and a peptide.

7. The radiopharmaceutical solution according to any of the preceding claims, wherein said complexing agent is selected from the group consisting of EDTMP, antibody-conjugated-DOTA, or antibody-conjugated-TCMC.

8. The radiopharmaceutical according to any of the preceding claims, wherein the radioactivity is 100 kBq to 100 MBq.

9. A kit comprising
• a first vial comprising a radiopharmaceutical solution according to any of the preceding claims, and
• a second vial comprising a neutralizing solution to adjust pH and/or isotonicity of the radiopharmaceutical solution prior to administration to a patient.

10. A kit comprising
• a first vial comprising a ²²⁴Ra solution;
• a second vial comprising a complexing agent selected from the group consisting of acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, and antibody-conjugated-TCMC,
wherein the complexing agent is capable of complexing a daughter nuclide of ²²⁴Ra, such as ²¹²Pb, and wherein the complexing agent does not complex ²²⁴Ra in the pharmaceutical solution; and
• optionally, instructions for mixing the first vial and the second vial, thereby forming a pharmaceutical composition ready to be administered to a patient 1 minute to 12 hours after mixing.

11. The kit according to claims 9-10, or the radiopharmaceutical solution according to any of claims 1-8 for use as a medicament.

12. The radiopharmaceutical solution according to any of claims 1-8 for use in the treatment of skeletal disease.

13. The radiopharmaceutical solution for use according to claim 12, wherein the skeletal disease is selected from the group consisting of skeletal metastases from cancers to the breast, prostate, kidneys, lung, bone, or multiple myeloma, or non-cancerous diseases causing undesired calcification including ankylosing spondylitis.

14. The radiopharmaceutical solution for use according to any of claims 11-13, wherein the solution is administered at a dose in the range 50-150 kBq per kg of bodyweight, such as 50-100 kBq per kg of bodyweight.

15. A method for providing a radiopharmaceutical solution according to any of claims 1-8, the method comprising:
a) providing a first solution wherein the amount of ²²⁴Ra and ²¹²Pb is in radioactive equilibrium;
b) providing a second solution comprising a complexing agent that is selected from the group consisting of acyclic chelators, cyclic chelators, cryptands, crown ethers, porphyrins or cyclic or noncyclic polyphosphonates, DOTMP, EDTMP, bisphosphonate, pamidronate conjugated to DOTA, pamidronate conjugated to TCMC, antibody-conjugated-DOTA, and antibody-conjugated-TCMC, wherein the complexing agent is capable of complexing a daughter nuclide of ²²⁴Ra, such as ²¹²Pb, and wherein the complexing agent does not complex ²²⁴Ra; and
c) mixing the first composition and the second composition, thereby providing a pharmaceutical composition according to any of claim 1-8.

## Patentansprüche

1. Radiopharmazeutische Lösung, die unkomplexiertes ²²⁴Ra und Komplexe zwischen einem Komplexiermittel und ²¹²Pb umfasst;
wobei das Komplexiermittel aus der Gruppe ausgewählt ist, die aus acyclischen Chelatbildnern, cyclischen Chelatbildnern, Kryptanden, Kronenethern, Porphyrinen oder cyclischen oder nichtcyclischen Polyphosphonaten, DOTMP, EDTMP, Bisphosphonat, Pamidronat konjugiert zu DOTA, Pamidronat konjugiert zu TCMC, TCMC, DOTA, p-SCN-Bn-DOTA, p-SCN-Bn-TCMC, Antikörper-konjugiertem-DOTA und Antikörper-konjugiertem-TCMC besteht.

2. Radiopharmazeutische Lösung, die ²²⁴Ra, ²¹²Pb und ein Komplexiermittel umfasst, das aus der Gruppe ausgewählt ist, die aus EDTMP, Antikörper-konjugiertem-DOTA oder Antikörper-konjugiertem-TCMC besteht.

3. Radiopharmazeutische Lösung nach Anspruch 1 oder 2, wobei das Komplexiermittel dazu in der Lage ist, zumindest ²¹²Pb zu komplexieren.

4. Radiopharmazeutische Lösung nach einem der vorhergehenden Ansprüche, wobei das Komplexiermittel dazu in der Lage ist, ein Tochternuklid von ²²⁴Ra, wie zum Beispiel ²¹²Pb, in der pharmazeutischen Lösung zu komplexieren.

5. Radiopharmazeutische Lösung nach einem der vorhergehenden Ansprüche, wobei das Komplexiermittel ²²⁴Ra in der pharmazeutischen Lösung nicht komplexiert.

6. Radiopharmazeutische Lösung nach einem der vorhergehenden Ansprüche, wobei das Komplexiermittel zu einer Verbindung konjugiert ist, die aus der Gruppe ausgewählt ist, die aus einem monoklonalen Antikörper, einem Vitamin, einem polyklonalen Antikörper, einem Antikörperfragment, einem synthetischen Protein und einem Peptid besteht.

7. Radiopharmazeutische Lösung nach einem der vorhergehenden Ansprüche, wobei das Komplexiermittel aus der Gruppe ausgewählt ist, die aus EDTMP, Antikörper-konjugiertem-DOTA oder Antikörper-konjugiertem-TCMC besteht.

8. Radiopharmazeutische Lösung nach einem der vorhergehenden Ansprüche, wobei die Radioaktivität 100 kBq bis 100 MBq beträgt.

9. Kit, umfassend
• eine erste Ampulle, die eine radiopharmazeutische Lösung nach einem der vorhergehenden Ansprüche umfasst, und
• eine zweite Ampulle, die eine neutralisierende Lösung zum Anpassen von pH und/oder Isotonizität der radiopharmazeutischen Lösung vor der Verabreichung an einen Patienten umfasst.

10. Kit, umfassend
• eine erste Ampulle, die eine ²²⁴Ra-Lösung umfasst;
• eine zweite Ampulle, die ein Komplexiermittel umfasst, das aus der Gruppe ausgewählt ist, die aus acyclischen Chelatbildnern, cyclischen Chelatbildnern, Kryptanden, Kronenethern, Porphyrinen oder cyclischen oder nichtcyclischen Polyphosphonaten, DOTMP, EDTMP, Bisphosphonat, Pamidronat konjugiert zu DOTA, Pamidronat konjugiert zu TCMC, Antikörper-konjugiertem-DOTA und Antikörper-konjugiertem-TCMC besteht, wobei das Komplexiermittel dazu in der Lage ist, ein Tochternuklid von ²²⁴Ra, wie zum Beispiel ²¹²Pb, zu komplexieren, und wobei das Komplexiermittel ²²⁴Ra in der pharmazeutischen Lösung nicht komplexiert; und
• optional Anweisungen zum Mischen der ersten Ampulle und der zweiten Ampulle, wodurch eine pharmazeutische Zusammensetzung gebildet wird, die bereit ist, einem Patienten 1 Minute bis 12 Stunden nach dem Mischen verabreicht zu werden.

11. Kit nach Anspruch 9-10 oder radiopharmazeutische Lösung nach einem der Ansprüche 1-8 zur Verwendung als Medikament.

12. Radiopharmazeutische Lösung nach einem der Ansprüche 1-8 zur Verwendung bei der Behandlung von Skeletterkrankung.

13. Radiopharmazeutische Lösung zur Verwendung nach Anspruch 12, wobei die Skeletterkrankung aus der Gruppe ausgewählt ist, die aus Skelettmetastasen von Brustkrebs, Prostatakrebs, Nierenkrebs, Lungenkrebs, Knochenkrebs oder multiplem Myelom oder Nichtkrebserkrankungen, die unerwünschte Verkalkung verursachen, darunter ankylosierende Spondylitis, besteht.

14. Radiopharmazeutische Lösung zur Verwendung nach einem der Ansprüche 11-13, wobei die Lösung in einer Dosis im Bereich von 50-150 kBq pro kg Körpergewicht verabreicht wird, wie zum Beispiel 50-100 kBq pro kg Körpergewicht.

15. Verfahren zum Bereitstellen einer radiopharmazeutischen Lösung nach einem der Ansprüche 1-8, wobei das Verfahren Folgendes umfasst:
a) Bereitstellen einer ersten Lösung, wobei die Menge an ²²⁴Ra und ²¹²Pb in radioaktivem Gleichgewicht ist;
b) Bereitstellen einer zweiten Lösung, die ein Komplexiermittel umfasst, das aus der Gruppe ausgewählt ist, die aus acyclischen Chelatbildnern, cyclischen Chelatbildnern, Kryptanden, Kronenethern, Porphyrinen oder cyclischen oder nichtcyclischen Polyphosphonaten, DOTMP, EDTMP, Bisphosphonat, Pamidronat konjugiert zu DOTA, Pamidronat konjugiert zu TCMC, Antikörper-konjugiertem-DOTA und Antikörper-konjugiertem-TCMC besteht, wobei das Komplexiermittel dazu in der Lage ist, ein Tochternuklid von ²²⁴Ra, wie zum Beispiel ²¹²Pb, zu komplexieren, und wobei das Komplexiermittel ²²⁴Ra nicht komplexiert; und
c) Mischen der ersten Zusammensetzung und der zweiten Zusammensetzung, wodurch eine pharmazeutische Zusammensetzung nach einem von Anspruch 1-8 bereitgestellt wird.

## Revendications

1. Solution radiopharmaceutique comprenant du ²²⁴Ra et des complexes entre un agent complexant et du ²¹²Pb ;
dans laquelle ledit agent complexant est choisi dans le groupe constitué de chélateurs acycliques, de chélateurs cycliques, de cryptands, d'éthers couronnes, de porphyrines ou de polyphosphonates cycliques ou non cycliques, de DOTMP, d'EDTMP, de bisphosphonate, de pamidronate conjugué à du DOTA ou de pamidronate conjugué à du TCMC, de TCMC, de DOTA, de p-SCN-Bn-DOTA, de p-SCN-Bn-TCMC, de DOTA conjugué à un anticorps et de TCMC conjugué à un anticorps.

2. Solution radiopharmaceutique comprenant du ²²⁴Ra, du ²¹²Pb et un agent complexant choisi dans le groupe constitué d'EDTMP, de DOTA conjugué à un anticorps ou de TCMC conjugué à un anticorps.

3. Solution radiopharmaceutique selon la revendication 1 ou 2, dans laquelle l'agent complexant est capable de complexer au moins du ²¹²Pb.

4. Solution radiopharmaceutique selon l'une quelconque des revendications précédentes, dans laquelle l'agent complexant est capable de complexer un nucléide fille de ²²⁴Ra, tel que ²¹²Pb, dans la solution pharmaceutique.

5. Solution radiopharmaceutique selon l'une quelconque des revendications précédentes, dans laquelle l'agent complexant ne complexe pas le ²²⁴Ra dans la solution pharmaceutique.

6. Solution radiopharmaceutique selon l'une quelconque des revendications précédentes, dans laquelle l'agent complexant est conjugué à un composé choisi dans le groupe constitué d'un anticorps monoclonal, d'une vitamine, d'un anticorps polyclonal, d'un fragment d'anticorps, d'une protéine synthétique et d'un peptide.

7. Solution radiopharmaceutique selon l'une quelconque des revendications précédentes, dans laquelle ledit agent complexant est choisi dans le groupe constitué d'EDTMP, de DOTA conjugué à un anticorps ou de TCMC conjugué à un anticorps.

8. Produit radiopharmaceutique selon l'une quelconque des revendications précédentes, dans lequel la radioactivité est comprise entre 100 kBq et 100 MBq.

9. Kit comprenant
• un premier flacon comprenant une solution radiopharmaceutique selon l'une quelconque des revendications précédentes, et
• un second flacon comprenant une solution de neutralisation pour ajuster le pH et/ou l'isotonicité de la solution radiopharmaceutique avant son administration à un patient.

10. Kit comprenant
• un premier flacon comprenant une solution de ²²⁴Ra ;
• un second flacon comprenant un agent complexant choisi dans le groupe constitué de chélateurs acycliques, de chélateurs cycliques, de cryptands, d'éthers couronnes, de porphyrines ou de polyphosphonates cycliques ou non cycliques, de DOTMP, d'EDTMP, de bisphosphonate, de pamidronate conjugué à du DOTA, de pamidronate conjugué à du TCMC, de DOTA conjugué à un anticorps et de TCMC conjugué à un anticorps,
dans lequel l'agent complexant est capable de complexer un nucléide fille de ²²⁴Ra, tel que ²¹²Pb, et dans lequel l'agent complexant ne complexe pas le ²²⁴Ra dans la solution pharmaceutique ; et
• éventuellement, des instructions pour mélanger le premier flacon et le second flacon, formant ainsi une composition pharmaceutique prête à être administrée à un patient 1 minute à 12 heures après le mélange.

11. Kit selon les revendications 9 et 10 ou solution radiopharmaceutique selon l'une quelconque des revendications 1 à 8 destiné(e) à être utilisé(e) en tant que médicament.

12. Solution radiopharmaceutique selon l'une quelconque des revendications 1 à 8, destinée à être utilisée dans le traitement d'une maladie du squelette.

13. Solution radiopharmaceutique destinée à être utilisée selon la revendication 12, dans laquelle la maladie du squelette est choisie dans le groupe constitué de métastases osseuses provenant de cancers du sein, de la prostate, des reins, des poumons, des os ou d'un myélome multiple ou de maladies non cancéreuses provoquant une calcification indésirable, notamment la spondylarthrite ankylosante.

14. Solution radiopharmaceutique destinée à être utilisée selon l'une quelconque des revendications 11 à 13, dans laquelle la solution est administrée à une dose située dans la plage allant de 50 à 150 kBq par kg de poids corporel, telle que 50 à 100 kBq par kg de poids corporel.

15. Procédé de fourniture d'une solution radiopharmaceutique selon l'une quelconque des revendications 1 à 8, le procédé comprenant :
a) la fourniture d'une première solution dans lequel la quantité de ²²⁴Ra et de ²¹²Pb est en équilibre radioactif ;
b) la fourniture d'une seconde solution comprenant un agent complexant choisi dans le groupe constitué de chélateurs acycliques, de chélateurs cycliques, de cryptands, d'éthers couronnes, de porphyrines ou de polyphosphonates cycliques ou non cycliques, de DOTMP, d'EDTMP, de bisphosphonate, de pamidronate conjugué à du DOTA, de pamidronate conjugué à du TCMC, de DOTA conjugué à un anticorps et de TCMC conjugué à un anticorps, dans lequel l'agent complexant est capable de complexer un nucléide fille de ²²⁴Ra, tel que ²¹²Pb, et dans lequel l'agent complexant ne complexe pas le ²²⁴Ra ; et
c) le mélange de la première composition et de la seconde composition, fournissant ainsi une composition pharmaceutique selon l'une quelconque des revendications 1 à 8.
